# EUROPEAN PATENT APPLICATION

(11) **EP 4 726 009 A1**
(43) Date of publication of application: **15.04.2026**
(21) Application number: 25203754.4
(22) Date of filing: 22.09.2025
(51) Int. Cl.: C09K 11/06, C07D 495/04, H10K 85/60

(54) **NOVEL ORGANIC COMPOUND, PHOTODIODE INCLUDING SAME, ELECTRONIC APPARATUS INCLUDING SAME PHOTODIODE**

(30) Priority: 11.10.2024 KR 20240138140; 18.11.2024 KR 20240163914; 04.08.2025 KR 20250106714
(71) Applicant: SFC Co., Ltd., Cheongju-si, Chungcheongbuk-do 28122 (KR)
(72) Inventor: KIM, Sung Woo, 28122 Cheongju-si (KR); KWON, Dae Ryeon, 28122 Cheongju-si (KR); PARK, Jong Beom, 28122 Cheongju-si (KR); PARK, Keun Hyeong, 28122 Cheongju-si (KR); JU, Eun Hae, 28122 Cheongju-si (KR)
(74) Representative: Epping - Hermann - Fischer

(57) **Abstract**

Disclosed herein are an organic compound represented by Chemical Formula 1 and a photodiode, wherein the organic compound is available in a photoactive layer of the photodiode. Chemical Formula 1 is as described in the description.

## Description

### BACKGROUND OF THE INVENTION

### 1. Field of the Invention

The present disclosure relates to a novel organic compound, a photodiode including same, and an electronic apparatus including the same photodiode. More specifically, the present disclosure relates to a novel organic compound, a photodiode including same, and an electronic apparatus including the same photodiode, the organic compound enabling improved light absorption efficiency and external quantum efficiency (EQE) in a photodiode, which converts optical energy or optical signals into electrical energy or electrical signals, exhibiting high thermal stability (Td) to prevent easy molecular decomposition, and providing a low dark current density (current flowing when light is absent).

### 2. Description of the Related Art

A photodiode is a device that converts optical energy or an optical signal into electrical energy or an electrical signal. Examples of photodiodes include photovoltaic cells or solar cells that convert optical energy into electrical energy, and photodetectors or optical sensors that detect optical energy and convert it into electrical signals.

Recently, electronic apparatuses including photodiodes and organic light emitting diodes (OLEDs) have been actively developed using various materials. Light emitted from an OLED may be reflected by an object (e.g., a user's finger) in contact with the electronic apparatus and incident onto the photodiode. In such cases, the photodiode detects the incident light energy and converts it into an electrical signal, allowing the electronic apparatus to recognize the contact of the object. Photodiodes can be used, for example, in fingerprint recognition sensors.

With regard to related arts of such photodiodes, reference may be made to Korean Patent Publication No. 10-2024-0057525 A (published on May 3, 2024) that discloses a photodiode including a first electrode, a second electrode facing the first electrode, a photoactive layer disposed between the first and second electrodes, and a boron compound having a specific structure, and Korean Patent Publication No. 10-2024-0132258 A (published on September 3, 2024) that discloses an organic electronic device including an electrode, a counter electrode, and an organic photoactive layer disposed between the two electrodes, wherein the organic photoactive layer includes an organic compound containing a heterocyclic ring formed by fusion of two five-membered rings.

However, despite the development of various materials for manufacturing photodiodes in the prior art including the above references, there remains a continuous need for the development of photodiode materials that offer improved light absorption efficiency and external quantum efficiency (EQE) particularly in specific wavelength regions such as the red and/or green and/or near infrared (NIR) wavelength regions, exhibit high thermal stability (Td) to resist molecular decomposition, and simultaneously demonstrate low dark current density (current flowing in the absence of light irradiation), as well as photodiodes comprising such materials.

### Related Art Document

Korean Patent Publication No. 10-2024-0057525 A (May 03, 2024)
Korean Patent Publication No. 10-2024-0132258 A (September 03, 2024)

### SUMMARY OF THE INVENTION

Accordingly, an aspect of the present disclosure is to provide an organic compound that can be used as a material in the photoactive layer of an organic photodiode and which exhibits improved light absorption efficiency and external quantum efficiency (EQE) in the red and/or green and/or near infrared(NIR) wavelength regions, high thermal stability (Td) to resist molecular decomposition, and a low dark current density (current flowing in the absence of light irradiation).

Another aspect of the present disclosure is to provide an organic photodiode with high light absorption efficiency, which includes the above organic compound.

In order to achieve the goals, the present disclosure provides an organic compound represented by the following Chemical Formula 1:

[Chemical Formula 1] A₁(L₁)ₘ-D-(L₂)ₙ-A₂

wherein,
L₁ and L₂, which are same and different, are each independently are any one selected from a single bond, a substituted or unsubstituted arylene of 6 to 18 carbon atoms, a substituted or unsubstituted aliphatic hydrocarbon ring-fused arylene of 8 to 20 carbon atoms, and a substituted or unsubstituted heteroarylene of 2 to 10 carbon atoms,
m and n, which are same or different, are each independently an integer of 1 or 2,
wherein when m is 2, the corresponding L₁'s are same or different, and
when n is 2, the corresponding L₂'s are same or different,
D is a moiety represented by the following Structural Formula 2,
A₁ and A₂, which are same or different, are moieties each represented by the following Structural Formula 3: 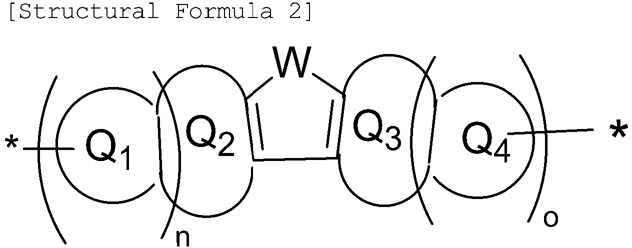 in Structural Formula 2,
   W is NR₁ or CR₂R₃,
   Q1 to Q4 rings, which are same or different, are each independently any one selected from a substituted or unsubstituted aromatic hydrocarbon ring of 6 to 50 carbon atoms and a substituted or unsubstituted heteroaromatic ring of 2 to 50 carbon atoms,
   n and o, which are same or different, are each independently an integer of 0 to 2,
   wherein when n is 2, the corresponding Q1 rings are same or different, and
   when o is 2, the corresponding Q4 rings are same or different,
   R₁ is any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted aromatic hydrocarbon ring-fused cycloalkyl of 7 to 30 carbon atoms, a substituted or unsubstituted aliphatic hydrocarbon ring-fused aryl of 8 to 30 carbon atoms, and a substituted or unsubstituted heteroaryl of 2 to 50 carbon atoms,
   R₂ and R₃, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted aromatic hydrocarbon ring-fused cycloalkyl of 7 to 30 carbon atoms, and a substituted or unsubstituted aliphatic hydrocarbon ring-fused aryl of 8 to 30 carbon atoms and may be connected to each other to form a ring, 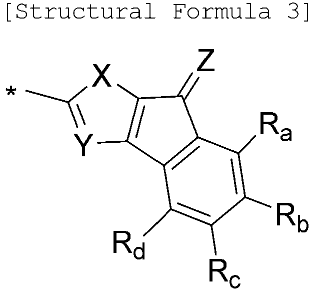 in Structural Formula 3,
      X is S or O,
      Y is any one selected from CH, CD, and N,
      Z is O or malononitrile(=C(CN)₂),
      Ra to Rd, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted amine of 0 to 40 carbon atoms, a substituted or unsubstituted silyl of 0 to 40 carbon atoms, a nitro, a cyano, and a halogen,
      in Structural Formulas 2 and 3, "*" means a bonding site to which the linker L₁ or L₂ in Chemical Formula 1 is bonded,
      wherein the term "substituted" in the expression "a substituted or unsubstituted" used for the compounds of Chemical Formula 1 and Structural Formulas 2 and 3 means having at least one substituent selected from the group consisting of a deuterium atom, a tritium atom, a cyano, a halogen, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.

Furthermore, the present disclosure provides a photodiode including: a first electrode; a second electrode facing the first electrode; and a photoactive layer disposed between the first electrode and the second electrode, wherein the photoactive layer includes the organic compound represented by Chemical Formula 1.

The organic compound represented by Chemical Formula 1 according to the present disclosure exhibits improved light absorption efficiency and external quantum efficiency (EQE) in the central region of the red and/or green and/or near infrared(NIR) wavelength range. In addition, the organic compound has high thermal stability (Td), which prevents easy decomposition of the molecule, and a low dark current density (i.e., the current that flows in the absence of light irradiation). Therefore, a photodiode including the organic compound can exhibit excellent device characteristics, including high light absorption efficiency.

### BRIEF DESCRIPTION OF THE DRAWINGS

FIGURE is a schematic diagram of a photodiode according to an embodiment of the present disclosure.

### DETAILED DESCRIPTION

Below, a detailed description will be given of the present disclosure. In each drawing of the present disclosure, sizes or scales of components may be enlarged or reduced from their actual sizes or scales for better illustration, and known components may not be depicted therein to clearly show features of the present disclosure. Therefore, the present disclosure is not limited to the drawings. When describing the principle of the embodiments of the present disclosure in detail, details of well-known functions and features may be omitted to avoid unnecessarily obscuring the presented embodiments.

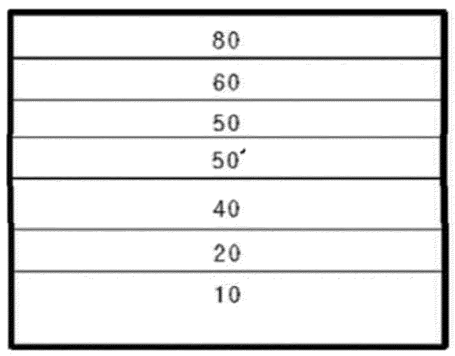

In the drawing, for convenience of description, sizes of components may be exaggerated for clarity. For example, since sizes and thicknesses of components in drawings are arbitrarily shown for convenience of description, the sizes and thicknesses are not limited thereto. Furthermore, throughout the description, the terms "on" and "over" are used to refer to the relative positioning, and mean not only that one component or layer is directly disposed on another component or layer but also that one component or layer is indirectly disposed on another component or layer with a further component or layer being interposed therebetween. Also, spatially relative terms, such as "below", "beneath", "lower", and "between" may be used herein for ease of description to refer to the relative positioning.

Throughout the specification, when a portion may "include" a certain constituent element, unless explicitly described to the contrary, it may not be construed to exclude another constituent element but may be construed to further include other constituent elements. Further, throughout the specification, the word "on" means positioning on or below the object portion, but does not essentially mean positioning on the lower side of the object portion based on a gravity direction.

The present disclosure provides an organic compound represented by the following Chemical Formula 1:

[Chemical Formula 1] A₁-(L₁)ₘ-D-(L2)ₙ-A₂

wherein,
L₁ and L₂, which are same and different, are each independently are any one selected from a single bond, a substituted or unsubstituted arylene of 6 to 18 carbon atoms, a substituted or unsubstituted aliphatic hydrocarbon ring-fused arylene of 8 to 20 carbon atoms, and a substituted or unsubstituted heteroarylene of 2 to 10 carbon atoms,
m and n, which are same or different, are each independently an integer of 1 or 2,
wherein when m is 2, the corresponding L₁'s are same or different, and
when n is 2, the corresponding L₂'s are same or different,
D is a moiety represented by the following Structural Formula 2,
A₁ and A₂, which are same or different, are moieties each represented by the following Structural Formula 3: 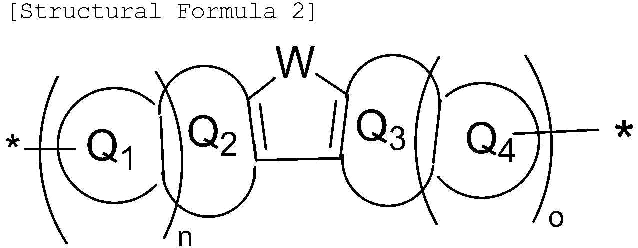 in Structural Formula 2,
   W is NR₁ or CR₂R₃,
   Q1 to Q4 rings, which are same or different, are each independently any one selected from a substituted or unsubstituted aromatic hydrocarbon ring of 6 to 50 carbon atoms and a substituted or unsubstituted heteroaromatic ring of 2 to 50 carbon atoms,
   n and o, which are same or different, are each independently an integer of 0 to 2,
   wherein when n is 2, the corresponding Q1 rings are same or different, and
   when o is 2, the corresponding Q4 rings are same or different,
   R₁ is any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted aromatic hydrocarbon ring-fused cycloalkyl of 7 to 30 carbon atoms, a substituted or unsubstituted aliphatic hydrocarbon ring-fused aryl of 8 to 30 carbon atoms, and a substituted or unsubstituted heteroaryl of 2 to 50 carbon atoms,
   R₂ and R₃, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted aromatic hydrocarbon ring-fused cycloalkyl of 7 to 30 carbon atoms, and a substituted or unsubstituted aliphatic hydrocarbon ring-fused aryl of 8 to 30 carbon atoms and may be connected to each other to form a ring, 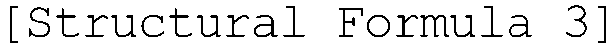 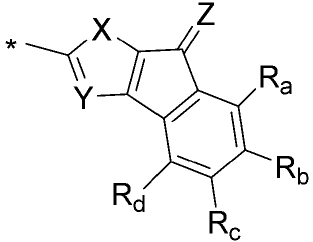 in Structural Formula 3,
      X is S or O,
      Y is any one selected from CH, CD, and N,
      Z is O or malononitrile(=C(CN)₂),
      Ra to Rd, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted amine of 0 to 40 carbon atoms, a substituted or unsubstituted silyl of 0 to 40 carbon atoms, a nitro, a cyano, and a halogen,
      in Structural Formulas 2 and 3, "*" means a bonding site to which the linker L₁ or L₂ in Chemical Formula 1 is bonded,
      wherein the term "substituted" in the expression "a substituted or unsubstituted" used for the compounds of Chemical Formula 1 and Structural Formulas 2 and 3 means having at least one substituent selected from the group consisting of a deuterium atom, a tritium atom, a cyano, a halogen, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.

The expression indicating the number of carbon atoms, such as "a substituted or unsubstituted alkyl of 1 to 30 carbon atoms", "a substituted or unsubstituted aryl of 6 to 50 carbon atoms", etc. means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical derived from an aromatic hydrocarbon by removing one hydrogen that is bonded to the aromatic hydrocarbon. When the aryl group is substituted, the substituents may be fused with one another to form an additional ring. The aryl group may also include an organic radical obtained by the removal of a single hydrogen atom from an arene ring formed by the fusion of two arene rings.

Concrete examples of the aryl include aromatic radical groups such as phenyl, o-biphenyl, m-biphenyl, p-biphenyl, o-terphenyl, m-terphenyl, p-terphenyl, naphthyl, anthryl, phenanthryl, pyrenyl, indenyl, fluorenyl, tetrahydronaphthyl, perylenyl, chrysenyl, naphthacenyl, fluoranthenyl, and triphenylenyl, but are not limited thereto. The aryl group may also include organic radicals obtained by the removal of one hydrogen atom from a fused arene ring formed by two arene rings, such as a fluorene ring fused with a phenylene ring or a fluorene ring fused with a phenanthrene ring.

In addition, at least one hydrogen atom on the aryl group may be substituted by a deuterium atom, a tritium atom, a halogen atom, a hydroxy, a nitro, a cyano, a silyl, an amino, a germanium, an amidino, a hydrazino, a hydrazone, a carboxyl, a sulfonic acid, a phosphoric acid, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, or an alkyl heteroaryl of 3 to 24 carbon atoms.

As used herein, the term "aromatic hydrocarbon ring" refers to an aromatic ring composed of carbon and hydrogen atoms and the term "aliphatic hydrocarbon ring" refers to a hydrocarbon ring that is composed of carbon and hydrogen atoms, but does not belong to the aromatic hydrocarbon rings. Particularly, the aliphatic hydrocarbon ring may have a bonding structure of the sp³ orbital for at least 30% of the carbon atoms as ring members, with 0 to 3 double and/or triple bonds within the ring. More particularly, the aliphatic hydrocarbon ring may have a bonding structure of the sp³ orbital for at least 50% of the carbon atoms as ring members, with 0 to 2 double and/or triple bonds within the ring.

The term "aliphatic hydrocarbon ring-fused aryl", as used herein, refers to a cyclic radical in which two adjacent carbon atoms as ring members of an aliphatic hydrocarbon ring and two adjacent carbon atoms as ring members of an aryl are fused with each other to share one double bond therebetween, with non-aromaticity across the molecule. Specific examples include, but are not limited to, tetrahydronaphthyl, tetrahydrobenzocycloheptene, tetrahydrophenanthrene, tetrahydroanthracenyl, and octahydrotriphenylene.

The substituent "heteroaryl", used in the compound of the present disclosure, means a hetero aromatic radical of 2 to 24 carbon atoms, bearing as ring member(s) one to three heteroatoms selected from among N, O, P, Si, S, Ge, Se, and Te. In the aromatic radical, two or more rings may be fused. One or more hydrogen atoms on the heteroaryl may be substituted by the same substituents as on the aryl.

Concrete examples of the heteroaryl include thiophenyl, furanyl, pyrrolyl, imidazolyl, thiazolyl, oxazolyl, oxadiazolyl, triazolyl, pyridinyl, bipyridinyl, pyrimidinyl, triazinyl, triazolyl, acridinyl, carbazolyl, acenaphthoquinoxalinyl, indenoquinazolinyl, indenoisoquinolinyl, indenoquinolinyl, pyridoindolyl, pyridazinyl, pyrazinyl, quinolinyl, quinazolinyl, quinoxalinyl, phthalazinyl, pyridopyrimidinyl, pyridopyrazinyl, pyrazinopyrazinyl, isoquinolinyl, indolyl, carbazolyl, benzoxazolyl, benzimidazolyl, benzothiazolyl, benzocarbazolyl, benzofuranyl, benzothiophenyl, benzoselenophenyl, dibenzothiophenyl, dibenzofuranyl, dibenzoselenophenyl, phenanthrolinyl, thiazolinyl, isoxazolyl, oxadiazolyl, thiadiazolyl, benzothiazolyl, phenoxazinyl, phenothiazinyl, azadibenzofuranyl, azadibenzothiophenyl, azadibenzoselenophenyl, indolocarbazolyl, and the like, but are not limited thereto.

In addition, the term "heteroaromatic ring", as used herein, refers to an aromatic hydrocarbon ring bearing at least one heteroatom as an aromatic ring member. In the heteroaromatic ring, one to three carbon atoms of the aromatic hydrocarbon may be substituted by at least one selected particularly from N, O, P, Si, S, Ge, Se, and Te.

As used herein, the term "aliphatic hydrocarbon ring-fused heteroaryl" refers to the same cyclic substituent as in the aliphatic hydrocarbon ring-fused aryl, with the exception that a heteroaryl, instead of an aryl, is substituted. Specific examples include, but are not limited to, tetrahydroindole, tetrahydrobenzofuranyl, tetrahydrobenzothiophene, tetrahydrocarbazole, tetrahydrodibenzofuranyl, tetrahydrobenzothiophene, tetrahydroquinoline, and tetrahydroquinoxaline.

Furthermore, the term "heteroaromatic ring", as used herein, refers to an aromatic hydrocarbon ring bearing at least one heteroatom as an aromatic ring member. In the heteroaromatic ring, one to three carbon atoms of the aromatic hydrocarbon may be substituted by at least one selected particularly from N, O, P, Si, S, Ge, Se, and Te.

As used herein, the term "fused ring in which an aromatic hydrocarbon ring is fused with an aliphatic hydrocarbon ring" means a fused ring in which an aromatic hydrocarbon ring has two adjacent carbon atoms in common with an aliphatic hydrocarbon ring, as exemplified by a tetrahydronaphthalene ring, dihydroindene, and the like, in which the benzene ring shares two adjacent carbon atoms with the cyclohexane ring.

In the present disclosure, the term "fused ring with a heteroaromatic hydrocarbon ring and an aliphatic hydrocarbon ring fused to each other" refer to a cyclic radical in which two adjacent carbon atoms as ring members of an aromatic hydrocarbon ring and two adjacent carbon atoms as ring members of an aliphatic hydrocarbon ring are fused with each other to share two carbon atoms therebetween, as exemplified by hexahydrodibenzofuran in which a benzofuran ring and a cyclohexane ring are fused by sharing their respective two adjacent carbon atoms as ring members.

As used herein, the term "alkyl" refers to an alkane missing one hydrogen atom and includes linear or branched structures. Examples of the alkyl substituent useful in the present disclosure include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclo hexylmethyl, octyl, n- octyl, tert- octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like. At least one hydrogen atom of the alkyl may be substituted by the same substituent as in the aryl.

As used herein, the term "halogenated alkyl" refers to an alkyl with at least one hydrogen atom substituted by a halogen. Preferably, the halogen may be a fluorine atom.

The term "cyclo", as used in substituents of the compounds of the present disclosure, such as cycloalkyl, cycloalkoxy, etc., refers to a structure responsible for a mono- or polycyclic ring of saturated hydrocarbons within an alkyl radical, an alkoxy radical, etc. Concrete examples of cycloalkyl include cyclopropyl, cyclobutyl, cyclopentyl, cyclohexyl, methylcyclopentyl, methylcyclohexyl, ethylcyclopentyl, ethylcyclohexyl, adamantyl, dicyclopentadienyl, decahydronaphthyl, norbornyl, bornyl, isobornyl, and so on. One or more hydrogen atoms on the cycloalkyl may be substituted by the same substituents as on the aryl. This is true of the cycloalkoxy.

In the present disclosure, the term "heterocycloalkyl" refers to a cycloalkyl with at least one heteroatom substituted as a ring member for a carbon atom within the ring. Preferably, one to three carbon atoms within the ring may be substituted by at least one selected from N, O, P, S, Si, Ge, Se, and Te.

The term "aromatic hydrocarbon ring- or heteroaromatic ring-fused cycloalkyl", as used herein, refers to a cyclic radical in which two adjacent carbon atoms as ring members of an aromatic or heteroaromatic hydrocarbon ring and two adjacent carbon atoms as ring members of a cycloalkyl are fused with each other to share one double bond therebetween, with non-aromaticity across the molecule. Specific examples include tetrahydronaphthyl, tetrahydrophenanthrene, tetrahydroquinoline, tetrahydroquinoxaline, and cyclopentabenzofuran.

The term "aromatic hydrocarbon ring-fused heterocyclic alkyl" is same as the aromatic hydrocarbon ring-fused cycloalkyl, with the exception that at least one carbon atom within the cycloalkyl ring is substituted by a heteroatom, with non-aromaticity across the molecule. Preferably, one to three carbon atoms within the cycloalkyl ring are substituted by at least one selected from N, O, P, S, Si, Ge, Se, and Te. Specific examples of aromatic hydrocarbon ring-heterocyclic alkyl include hexahydrodibenzofuranyl, hexahydrocarbozole, hexahydrodibenzothiophene, and dihydrobenzodioxine.

As used herein, the "heteroaliphatic ring-fused aryl or heteroaryl" is same as the aliphatic hydrocarbon ring-fused aryl or heteroaryl, except for a heteroaliphatic ring substituted for the aliphatic hydrocarbon ring, with non-aromaticity across the molecule thereof. Specific examples include cromane, dihydropyranopyridine, thiocromane, dihydrobenzodioxine, dihydrothiopyranopyridine, and dihydropyranopyrimidine, but are not limited thereto.

As used herein, the term "heteroaliphatic ring" refers to an aliphatic hydrocarbon bearing at least one heteroatom as a ring member. Preferably, one to three carbon atoms within an aliphatic hydrocarbon are substituted by at least one heteroatom selected from N, O, and S.

The term "alkoxy", as used in the compounds of the present disclosure, refers to an alkyl or cycloalkyl singularly bonded to oxygen. Specific examples of the alkoxy include methoxy, ethoxy, propoxy, isobutoxy, sec-butoxy, pentoxy, iso-amyloxy, hexyloxy, cyclobutyloxy, cyclopentyloxy, adamantyloxy, dicyclopentyloxy, bornyloxy, isobornyloxy, and the like, but are not limited thereto. One or more hydrogen atoms on the alkoxy may be substituted by the same substituents as on the aryl.

Specific examples of the arylalkyl used in the compounds of the present disclosure include phenylmethyl (benzyl), phenylethyl, phenylpropyl, naphthylmethyl, naphthylethyl, and the like, but are not limited thereto. One or more hydrogen atoms on the arylalkyl may be substituted by the same substituents as on the aryl.

Specific examples of the alkylaryl used in the compound of the present disclosure include tolyl, xylenyl, dimethylnaphthyl, t-butylphenyl, t-butylnaphthyl, and t-butylphenanthryl, but are not limited thereto. One or more hydrogen atoms on the alkylaryl may be substituted by the same substituents as on the aryl.

As used herein, the term "alkenyl" refers to an alkyl substituent containing a carbon-carbon double bond between two carbon atoms and the term "alkynyl" refers to an alkyl substituent containing a carbon-carbon triple bond between two carbon atoms.

As used herein, the term "alkylene" refers to an organic radical regarded as derived from an alkane by removal two hydrogen atoms from one carbon atom for methylene or different carbon atoms for ethylene or higher, examples of which include, but are not limited to, methylene, ethylene, propylene, isopropylene, isobutylene, sec-butylene, tert-butylene, pentylene, iso-amylene, hexylene, and the like. One or more hydrogen atoms on the alkylene may be substituted by the same substituents as on the aryl.

In the present disclosure, the term "amine" is intended to encompass -NH₂, alkylamine, arylamine, alkylarylamine, arylheteroarylamine, heteroarylamine, and the like. The term "arylamine" refers to a functional group including -NH₂, in which one or two of the hydrogen atoms in -NH₂ are substituted by aryl; the term "alkylamine" to a functional group including -NH₂, in which one or two of the hydrogen atoms in -NH₂ are substituted by alkyl; the term "alkylarylamine" to a functional group including -NH₂, in which one of the hydrogen atoms in -NH₂ is substituted by alkyl and the other hydrogen atom is substituted by aryl; the term "arylheteroarylamine to a functional group including -NH₂, in which one of the hydrogen atoms in -NH₂ is substituted by aryl and the other hydrogen atom is substituted by heteroaryl; and the term "heteroarylamine" to a functional group including -NH₂, in which one or two of the hydrogen atoms in -NH₂ are substituted by heteroaryl. Examples of the arylamine include a substituted or unsubstituted monoarylamine and a substituted or unsubstituted diarylamine. Such examples are true of the alkylamine and the heteroarylamine.

Here, each of the aryl radicals in the arylamine, heteroarylamine, and arylheteroarylamine may be a monocyclic or polycyclic one. Each of the heteroaryl radicals in the heteroarylamine, and arylheteroarylamine may be a mono- or polycyclic one.

The term "silyl" used in the compounds of the present disclosure is intended to encompass -SiH₃, alkylsilyl, arylsilyl, alkylarylsilyl, arylheteroarylsilyl, heteroarylsilyl, and the like. The arylsilyl refers to a functional group based on -SiH₃ in which at least one of the three hydrogen atoms bonded to the silicon atom is substituted by aryl; the alkylsilyl to a functional group based on -SiH₃ in which at least one of the three hydrogen atoms bonded to the silicon atom is substituted by alkyl; the alkylarylsilyl to a functional group based on -SiH₃ in which at least two of the three hydrogen atoms bonded to the silicon atom are substituted by alkyl and aryl, respectively, as exemplified by a silyl having one or two alkyl radicals and correspondingly two or one aryl radicals; the arylheteroarylsilyl to a functional group based on -SiH₃ in which at least two of the three hydrogen atoms bonded to the silicon atom are substituted by aryl and heteroaryl, as exemplified by a silyl having one or two aryl radicals and correspondingly two or one heteroaryl radical; and the heteroarylsilyl to a functional group based on -SiH₃ in which at least one of the three hydrogen atoms bonded to the silicon atom is substituted by heteroaryl. Examples of the arylsilyl include a substituted or unsubstituted monoarylsilyl, a substituted or unsubstituted diarylsilyl, and a substituted or unsubstituted triarylsilyl. Such examples are true of the alkylsilyl and the heteroarylsilyl.

Here, each of the aryl radicals in the arylsilyl, heteroarylsily, and arylheteroarylsilyl in the heteroarylamine may be a mono- or polycyclic aryl, and each of the heteroaryl radicals in the arylsilyl, heteroarylsilyl, and arylheteroarylsilyl may be a mono- or polycyclic heteroaryl.

Furthermore, specific examples of the silyl include trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl. One or more hydrogen atoms on the silyl may be substituted by the same substituents as on the aryl.

As used herein, the term "germanium" (or germyl or germane) is intended to encompass -GeH₃, alkyl germanium, aryl germanium, heteroaryl germanium, alkylaryl germanium, alkylheteroaryl germanium, and arylheteroaryl germanium and can be accounted for by the definitions for silyl radicals, with the exception that the silicone atom (Si) in the silyl radical is substituted by a germanium atom (Ge) .

Specific examples of the germanium radical include trimethylgermane, triethylgermane, triphenylgermane, trimethoxygermane, dimethoxyphenylgermane, diphenylmethylgermane, diphenylvinylgermane, methylcyclobutylgermane, and dimethylfurylgermane. One or more hydrogen atoms on the germanium radical may be substituted by the same substituents as on the aryl.

As used herein, the term "alkenyl" refers to an alkyl substituent containing a carbon-carbon double bond between two carbon atoms and the term "alkynyl" refers to an alkyl substituent containing a carbon-carbon triple bond between two carbon atoms.

In a more preferable embodiment of the present disclosure, the substituted or unsubstituted aromatic hydrocarbon ring-fused cycloalkyl of 7 to 30 carbon atoms may be a substituted or unsubstituted aromatic hydrocarbon ring-fused cycloalkyl of 9 to 20 carbon atoms

In a more preferable embodiment of the present disclosure, the substituted or unsubstituted heteroaromatic ring-fused cycloalkyl of 5 to 30 carbon atoms may be a substituted or unsubstituted heteroaromatic ring-fused cycloalkyl of 7 to 20 carbon atoms.

In a more preferable embodiment of the present disclosure, the substituted or unsubstituted aromatic hydrocarbon ring-fused heterocycloalkyl of 6 to 30 carbon atoms may be a substituted or unsubstituted aromatic hydrocarbon ring-fused heterocycloalkyl of 9 to 20 carbon atoms.

In a more preferable embodiment of the present disclosure, the substituted or unsubstituted aliphatic hydrocarbon ring-fused aryl of 8 to 30 carbon atoms may be a substituted or unsubstituted aliphatic hydrocarbon ring-fused aryl of 9 to 20 carbon atoms.

In a more preferable embodiment of the present disclosure, the substituted or unsubstituted aliphatic hydrocarbon ring-fused heteroaryl of 5 to 30 carbon atoms may be a substituted or unsubstituted aliphatic hydrocarbon ring-fused heteroaryl of 7 to 20 carbon atoms.

In a more preferable embodiment of the present disclosure, the substituted or unsubstituted heteroaliphatic ring-fused aryl of 6 to 30 carbon atoms may be a substituted or unsubstituted heteroaliphatic ring-fused aryl of 7 to 20 carbon atoms.

In a more preferable embodiment of the present disclosure, the substituted or unsubstituted heteroaliphatic ring-fused heteroaryl of 5 to 30 carbon atoms may be a substituted or unsubstituted heteroaliphatic ring-fused heteroaryl of 6 to 20 carbon atoms.

As used herein, the expression "R₃₁ to R₃₇ may be linked to the A₁ to A₃ ring moieties to additionally form a mono- or polycyclic aliphatic or aromatic ring" means that each substituent selected from R₃₁ to R₃₇ (i.e., each of the R₃₁ to R₃₇ substituents) may form an additional ring by removing one hydrogen radical from the substituent and one hydrogen radical from each of the rings A₁ to A₃ for the purpose of forming a mono- or polycyclic aliphatic or aromatic ring, and then linking them together. Likewise, in the expression "a linkage may be made between R₃₂ and R₃₃, between R₃₄ and R₃₅, and between R₃₆ and R₃₇ to additionally form a mono- or polycyclic aliphatic or aromatic ring," when, for example, R₃₂ and R₃₃ form a ring, it means that one hydrogen radical is removed from R₃₂ and one hydrogen radical is removed from R₃₃, and the two are then connected to additionally form a ring.

In a preferable embodiment, the substituent accounted for by the term "substituted" in the expression "substituted or unsubstituted" used for compounds of Chemical Formula 1 may be at least one selected from the group consisting of a deuterium atom, a tritium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 3 to 18 carbon atoms, an alkyl heteroaryl of 3 to 18 carbon atoms, an aromatic hydrocarbon ring-fused cycloalkyl of 9 to 20 carbon atoms, a heteroaromatic ring-fused cycloalkyl of 7 to 20 carbon atoms, an aromatic hydrocarbon ring-fused heterocycloalkyl of 9 to 20 carbon atoms, an aliphatic hydrocarbon ring-fused aryl of 9 to 20 carbon atoms, an aliphatic hydrocarbon ring-fused heteroaryl of 7 to 20 carbon atoms, an alkoxy of 1 to 12 carbon atoms, an amine of 1 to 18 carbon atoms, a silyl of 1 to 18 carbon atoms, a germanium of 1 to 18 carbon atoms, an aryloxy of 6 to 18 carbon atoms, and an arylthionyl of 6 to 18 carbon atoms. At least one hydrogen atom within each substituent may be substituted with a deuterium or tritium atom.

With the structure represented by Chemical Formula 1 in which the conjugated heterocyclic ring represented by Structural Formula 2, which accounts for the central portion, is bonded to a substituent "(L₁)m-A₁" at one end and a substituent "(L₂)n-A₂" at the other end, the compound of the present disclosure is useful as a material for a photoactive layer in a photodiode and offers characteristics including improved light absorption efficiency and external quantum efficiency (EQE) in the central regions of red and/or green and/or near infrared (NIR) wavelengths, high thermal stability (Td) to resist molecular decomposition, and low dark current density (current flowing in the absence of light irradiation).

In an embodiment, Q1 to Q4 rings in Structural Formula 2, which are same or different, may be each independently any one selected from a substituted or unsubstituted thiophene ring, a substituted or unsubstituted furan ring, a substituted or unsubstituted benzene ring, a substituted or unsubstituted benzothiophene ring, a substituted or unsubstituted benzofuran ring, and a substituted or unsubstituted indole ring. In this regard, the compound represented by Structural Formula 2 may be a compound represented by the following Structural Formulas 2a and 2b: 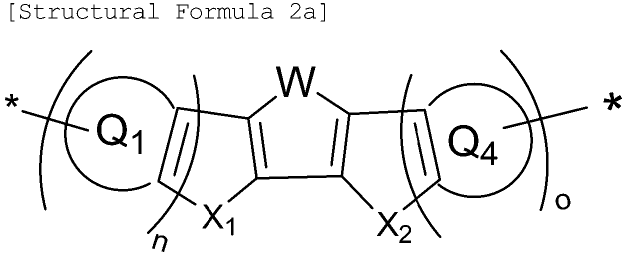 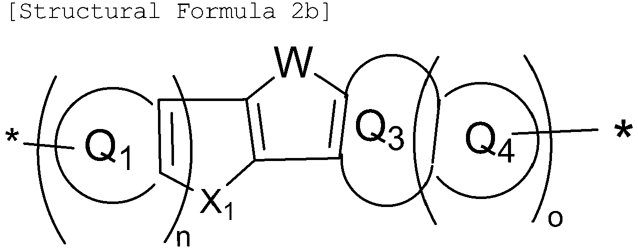 wherein,
X₁ and X₂, which are same or different, are each independently any one selected from S, O, and NR,
R is any one selected from a hydrogen atom, a deuterium atom, a tritium atom, a substituted or unsubstituted alkyl of 1 to 30 carbon atoms, a substituted or unsubstituted cycloalkyl of 3 to 30 carbon atoms, a substituted or unsubstituted aryl of 6 to 50 carbon atoms, a substituted or unsubstituted aliphatic hydrocarbon ring-fused aryl of 8 to 30 carbon atoms, and a substituted or unsubstituted heteroaryl of 2 to 50 carbon atoms,
the Q3 ring in Structural Formula 2b is a substituted or unsubstituted benzene ring, and
W, Q1, Q4, n, and o are each as defined in Structural Formula 2.

In an embodiment, Q1 to Q4 rings in Structural Formula 2, which are same or different, may be each independently any one selected from a substituted or unsubstituted thiophene ring, a substituted or unsubstituted furan ring, a substituted or unsubstituted benzene ring, a substituted or unsubstituted benzothiophene ring, a substituted or unsubstituted benzofuran ring, and a substituted or unsubstituted indole ring, and preferably, the Q2 and Q3 rings in Structural Formula 2 may each be a substituted or unsubstituted benzene ring.

In an embodiment, at least one hydrogen atom in Chemical Formula 1 may be substituted with a deuterium atom or a tritium atom.

In an embodiment, W in Structural Formula 2 may be NR₁, wherein R₁ may be any one substituent selected from a substituted or unsubstituted alkyl of 1 to 20 carbon atoms, a substituted or unsubstituted halogenated alkyl of 1 to 20 carbon atoms, a substituted or unsubstituted aryl of 6 to 20 carbon atoms, a substituted or unsubstituted cycloalkyl of 3 to 20 carbon atoms, a substituted or unsubstituted aromatic hydrocarbon ring-fused cycloalkyl of 8 to 20 carbon atoms, and a substituted or unsubstituted aliphatic hydrocarbon ring-fused aryl of 9 to 20 carbon atoms.

In an embodiment, o in Structural Formula 2 may be 0.

In an embodiment, the Q3 ring in Structural Formula 2 may be a substituted or unsubstituted thiophene ring or a substituted or unsubstituted furan ring.

In an embodiment, the compound represented by Chemical Formula 1 may be any one selected from the following Compounds 1 to 39, but with no limitations thereto:

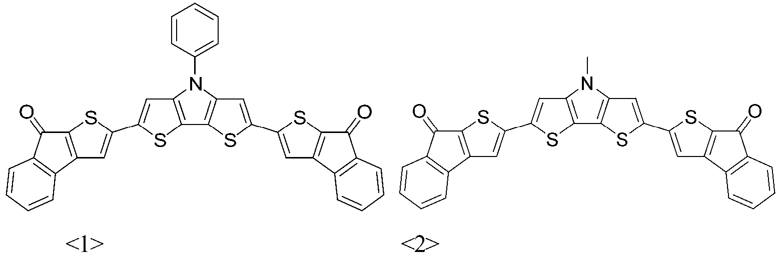

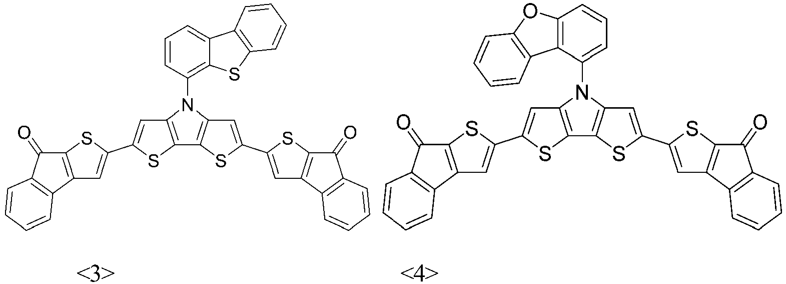

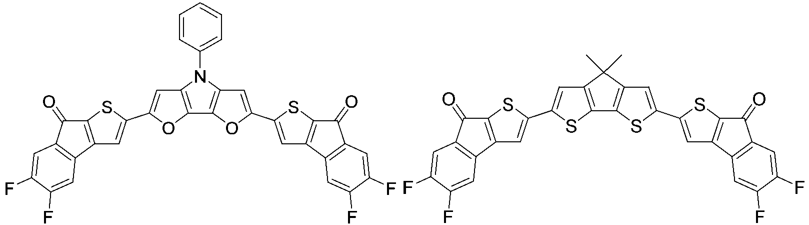

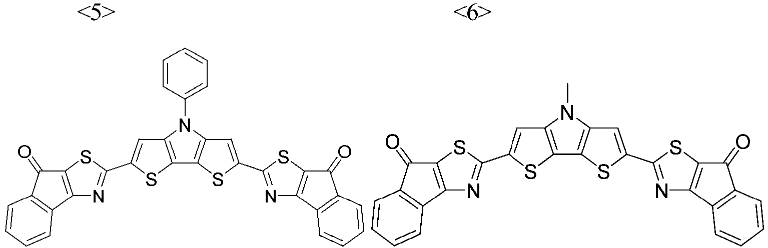

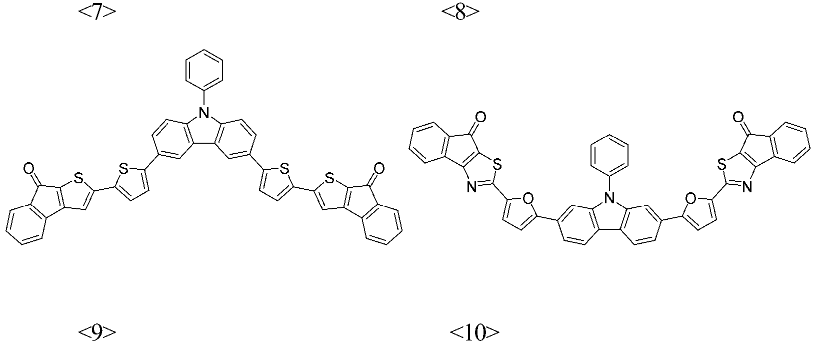

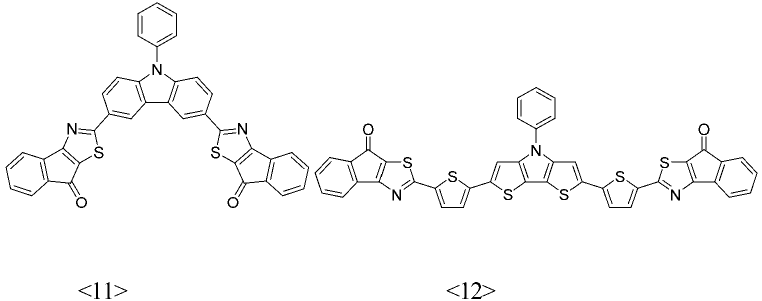

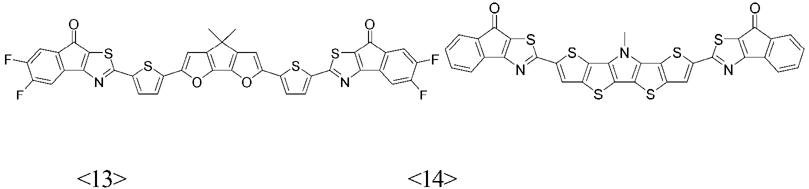

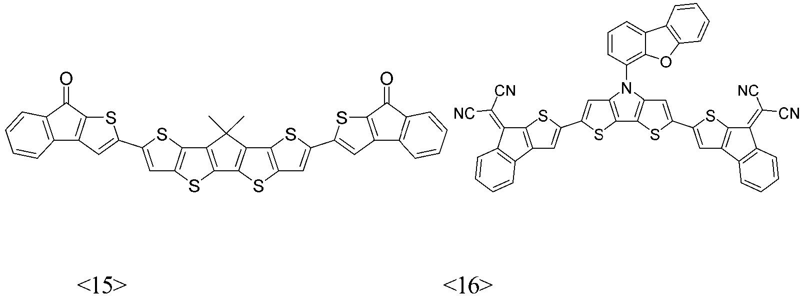

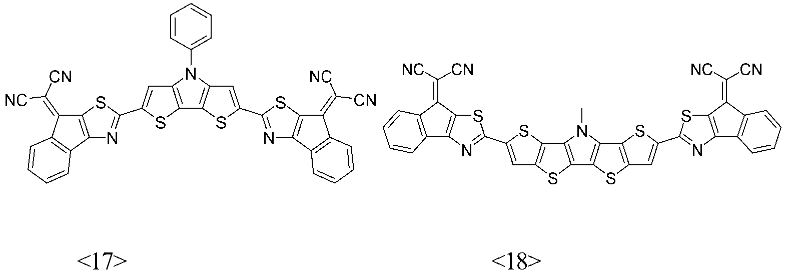

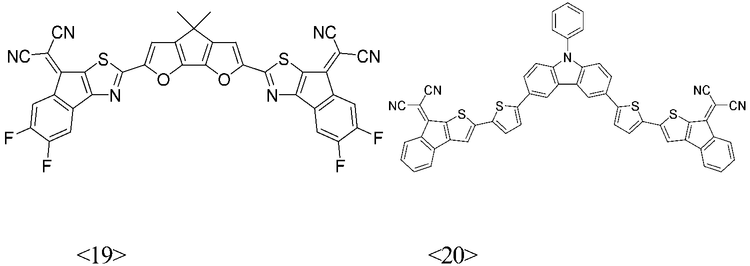

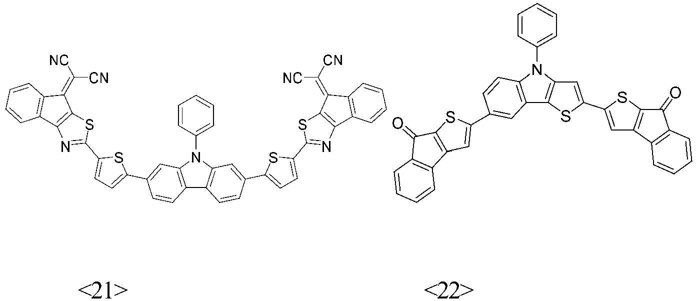

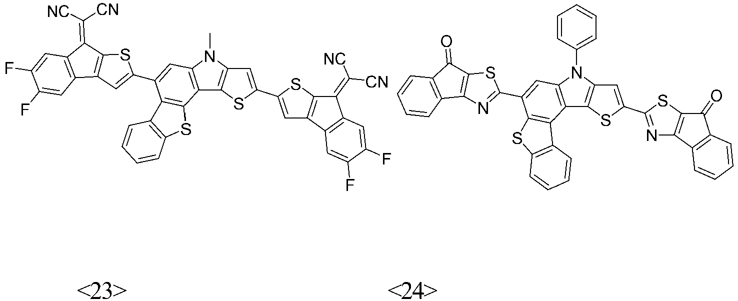

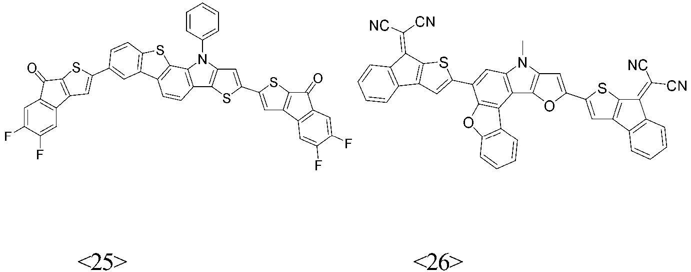

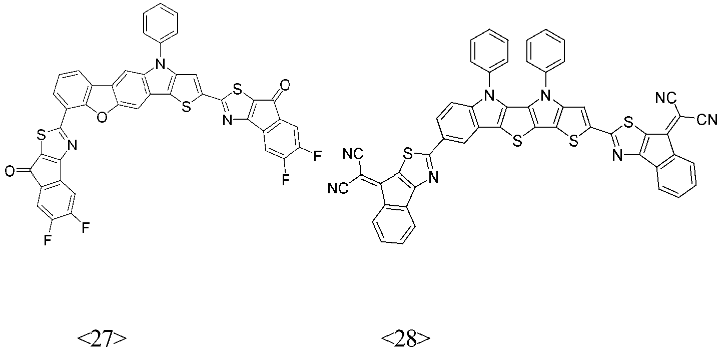

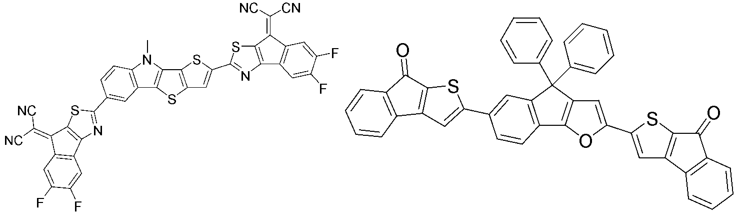

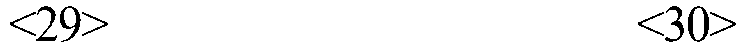

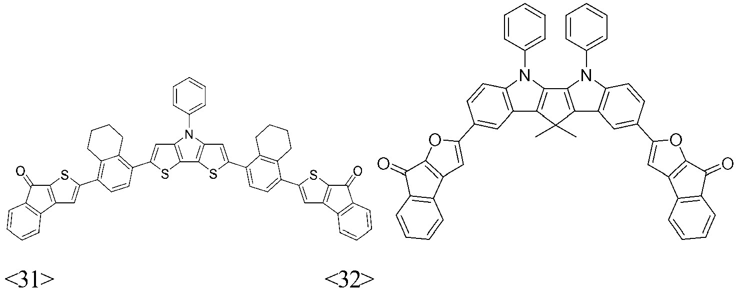

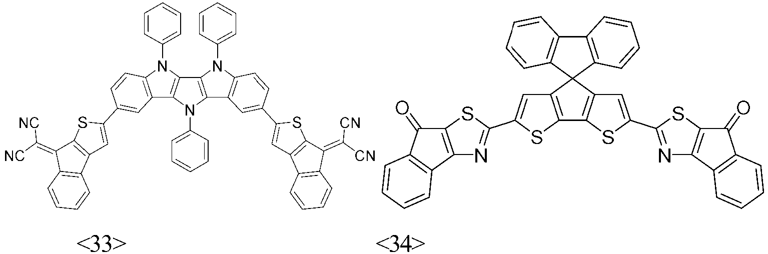

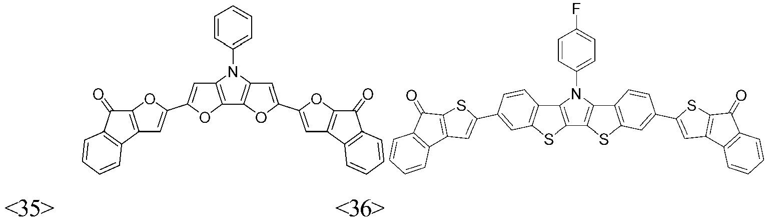

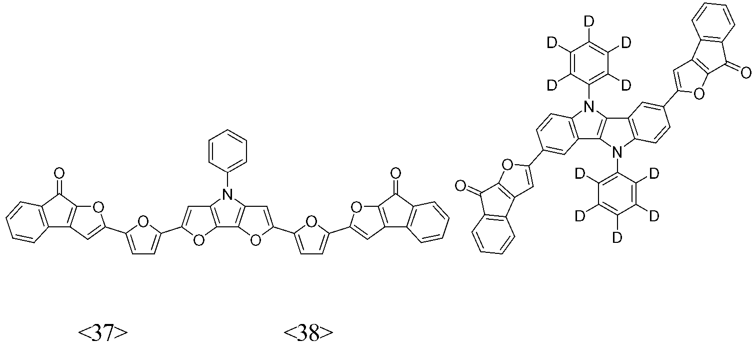

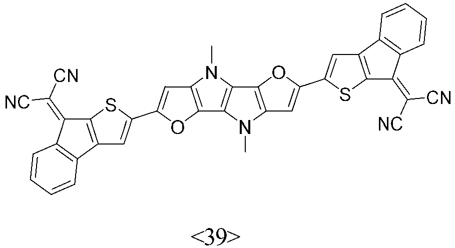

Throughout the description of the present disclosure, the phrase "(organic layer or emission layer) includes at least one organic compound" may be construed to mean that "(organic layer) may include a single organic compound species or two or more different species of organic compounds falling within the scope of the present disclosure".

According to the present disclosure, the compound represented by Chemical Formula 1 may be used as a compound in the photoactive layer of the photodiode. That is, the photodiode according to the present disclosure may include one or more of the compounds represented by Chemical Formula 1 as a material in the photoactive layer.

In an embodiment, the photodiode according to the present disclosure includes: a first electrode; a second electrode facing the first electrode; and a photoactive layer disposed between the first electrode and the second electrode, wherein the photoactive layer may include an organic compound represented by Chemical Formula 1.

In an embodiment, the first electrode may be an anode while the second electrode may be a cathode.

In this regard, the photodiode may further include a hole transport region disposed between the first electrode and the photoactive layer and further include an electron transport region between the photoactive layer and the second electrode. In this case, the photoactive layer may include a first layer adjacent to the hole transport region and a second layer adjacent to the electron transport region, and the organic compound represented by Chemical Formula 1 according to the present disclosure may be contained in the first layer.

In an embodiment, the maximum absorption wavelength of the organic compound may be about 450 nm to about 900 nm, and more preferably, the maximum absorption wavelength of the organic compound may be about 500 nm to about 800 nm.

The organic compound represented by Chemical Formula 1 may exhibit excellent light absorption efficiency within the above-described absorption wavelength range, for example, in the red or green or near infrared(NIR) region. Accordingly, a photodiode including the organic compound may have high light absorption efficiency with respect to light in the red or green or near infrared(NIR) wavelength range.

Here, the first layer may correspond to a P-type layer or an electron donor layer, and the second layer may correspond to an N-type layer or an electron acceptor layer.

In an embodiment, the first layer in the photoactive layer of the photodiode according to the present disclosure may be in direct contact with the hole transport region, and the second layer may be in direct contact with the electron transport region.

According to an embodiment, the hole transport region may include a hole injection layer, a hole transport layer, an emission auxiliary layer, an electron blocking layer, or any combination thereof. In addition, the electron transport region may include a buffer layer, a hole blocking layer, an electron control layer, an electron transport layer, an electron injection layer, or any combination thereof.

The present disclosure also provides an electronic apparatus including the photodiode according to the present disclosure. For example, the electronic apparatus may further include a thin film transistor electrically connected to the first electrode, and an emission layer disposed between the first electrode and the second electrode, the emission layer being non-overlapping with the photoactive layer.

For instance, the electronic apparatus may include the first electrode, the emission layer, and the second electrode, and further include: a hole transport region disposed between the first electrode and the emission layer and overlapping the emission layer; and an electron transport region disposed between the second electrode and the emission layer and overlapping the emission layer.

In addition, the electronic apparatus in the present disclosure may include an emission layer, wherein the emission layer is disposed between the first electrode and the second electrode, includes a dopant and a host, and is capable of emitting light.

Further, the hole transport region and the electron transport region may serve as common layers directly contacting and overlapping both the photoactive layer and the emission layer.

Moreover, the present disclosure provides an electronic device including the electronic apparatus according to the present disclosure. The electronic device may be, for example, a flat panel display, a curved display, a computer monitor, a medical monitor, a television, a billboard, indoor or outdoor lighting and/or signal light, a head-up display, a fully or partially transparent display, a flexible display, a rollable display, a foldable display, a stretchable display, a laser printer, a telephone, a mobile phone, a tablet, a phablet, a personal digital assistant (PDA), a wearable device, a laptop computer, a digital camera, a camcorder, a viewfinder, a microdisplay, a 3D display, a virtual reality or augmented reality display, a vehicle, a video wall comprising tiled multiple displays, a theater or stadium screen, a phototherapy device, or a signboard.

Below, with reference to the drawing, a description will be given of a photodiode according to an embodiment of the present disclosure.

FIGURE is a schematic view illustrating the structure of a photodiode according to an embodiment of the present disclosure.

As shown in FIGURE, the photodiode according to an embodiment of the present disclosure sequentially includes a first electrode (20), a hole transport layer (40), photoactive layers (50, 50'), an electron transport layer (60), and a second electrode (80), wherein the first electrode and the second electrode serve as an anode and a cathode, respectively, with the interposition of the hole transport layer (40) between the first electrode (anode) and the photoactive layers, and the electron transport layer (60) between the photoactive layer and the second electrode (cathode).

Furthermore, in the photodiode according to an embodiment of the present disclosure, the hole transport region (40) may include a hole injection layer and a hole transport layer, and an electron transport region (60) may include an electron transport layer and electron injection layer.

Reference is made to FIGURE with regard to the photodiode of the present disclosure and the fabrication method therefor.

First, a substrate (10) is coated with a first electrode material, i.e. anode material to form a first electrode (20). Preferable is a glass substrate or transparent plastic substrate that exhibits excellent transparency, surface smoothness, ease of handling, and waterproofness.

In addition, the substrate may be a flexible substrate and may include, for example, a plastic material having excellent heat resistance and durability, such as polyimide, polyethylene terephthalate (PET), polycarbonate, polyethylene naphthalate, polyarylate (PAR), polyetherimide, or any combination thereof.

Further, the first electrode (20) may be formed, for example, on the substrate by providing a first electrode material using a deposition method or a sputtering method. When the first electrode (20) is an anode, a high work function material that facilitates hole injection may be used as the first electrode material.

The first electrode (20) may be a reflective electrode, a semi-transparent electrode, or a transparent electrode. To form the first electrode as a transparent electrode, the first electrode material may include indium tin oxide (ITO), indium zinc oxide (IZO), tin oxide (SnO₂), zinc oxide (ZnO), or any combination thereof. Alternatively, to form the first electrode as a semi-transparent electrode or a reflective electrode, the first electrode material may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode may have a single-layer structure consisting of a single layer or a multi-layer structure including a plurality of layers. For example, the first electrode (20) may have a three-layer structure of ITO/Ag/ITO.

Subsequently, a hole transport region (40) is formed on the first electrode. The hole transport region (40) may have: i) a single-layer structure consisting of a single material; ii) a single-layer structure consisting of a single layer containing a plurality of different materials; or iii) a multi-layer structure including a plurality of layers containing a plurality of different materials.

For example, the hole transport region (40) may include a hole injection layer, a hole transport layer, an emission auxiliary layer, an electron blocking layer, or any combination thereof.

Here, the emission auxiliary layer is a layer that compensates for the optical resonance distance according to the wavelength of light emitted from the emission layer, thereby increasing light emission efficiency. The electron blocking layer is a layer that prevents electron leakage from the emission layer to the hole transport region (40). The above-described materials that can be included in the hole transport region (40) may also be included in the emission auxiliary layer and the electron blocking layer.

The hole transport region (40) may further include a charge-generating material to improve conductivity. The charge-generating material may be uniformly or non-uniformly dispersed within the hole transport region (40) (for example, in the form of a single layer consisting of a charge-generating material), and preferably, the charge-generating material may be a p-dopant.

For example, the LUMO energy level of the p-dopant may be -3.5 eV or lower.

According to one embodiment, the p-dopant may include a quinone derivative, a cyano group-containing compound, an element EL1- or element EL2-containing compound, or any combination thereof.

In one embodiment, the hole transport region (40) may have a multilayer structure such as: a hole injection layer/hole transport layer, a hole injection layer/hole transport layer/emission auxiliary layer, a hole injection layer/emission auxiliary layer, a hole transport layer/emission auxiliary layer, or a hole injection layer/hole transport layer/electron blocking layer, sequentially stacked from the first electrode (20).

Here, the hole transport region (40) may include one or more of the compounds HT1 to HT46, m-MTDATA, TDATA, 2-TNATA, NPB (NPD), β-NPB, TPD, Spiro-TPD, Spiro-NPB, methylated-NPB, TAPC, HMTPD, TCTA (4,4',4''-tris(N-carbazolyl)triphenylamine), PANI/DBSA (polyaniline/dodecylbenzenesulfonic acid), PEDOT/PSS (poly(3,4-ethylenedioxythiophene)/poly(4-styrenesulfonate)), PANI/CSA (polyaniline/camphor sulfonic acid), PANI/PSS (polyaniline/poly(4-styrenesulfonate)), or any combination thereof.

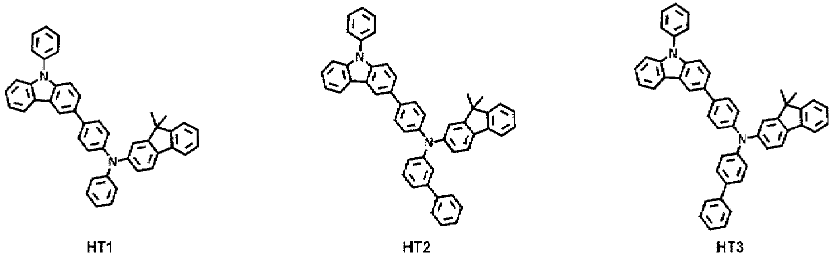

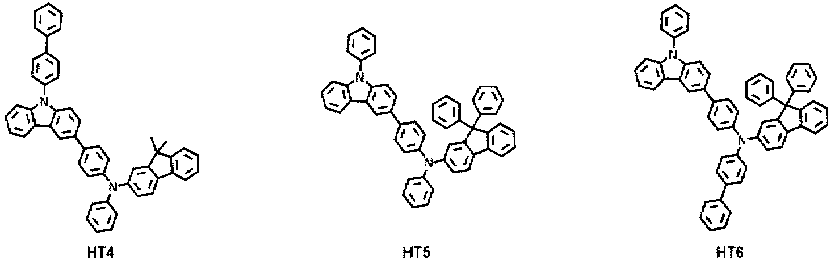

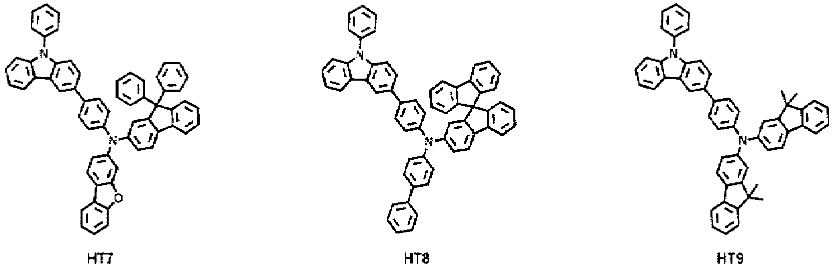

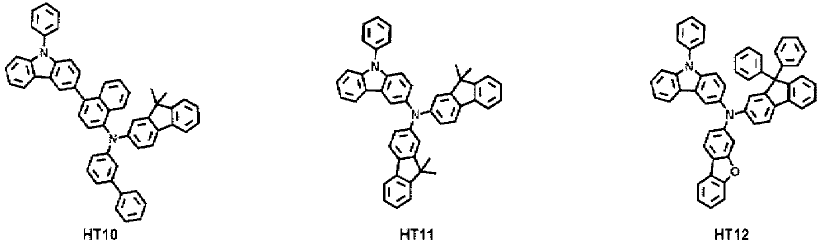

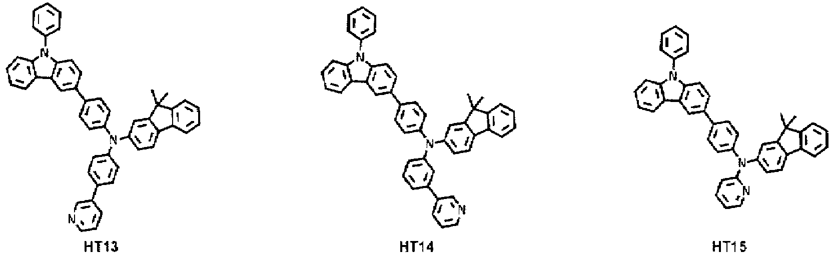

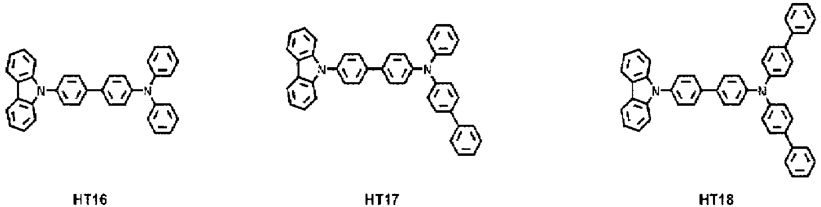

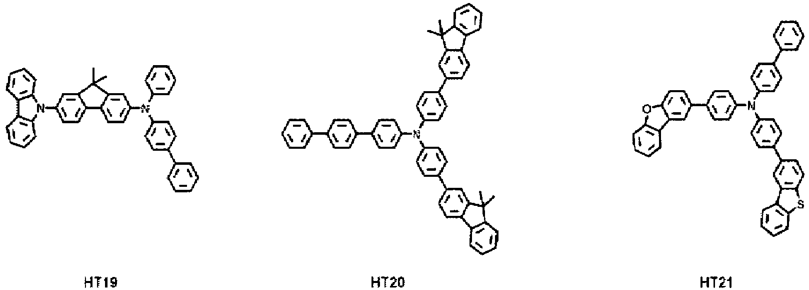

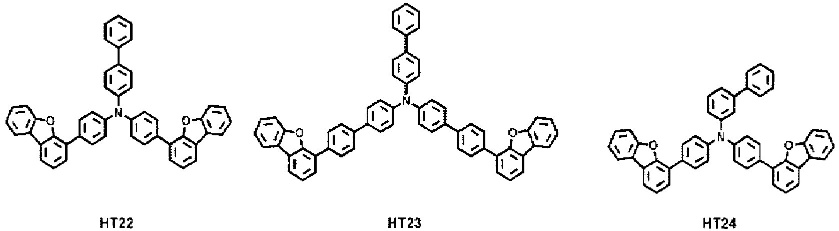

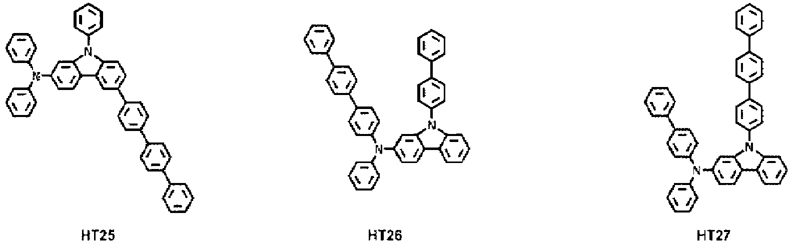

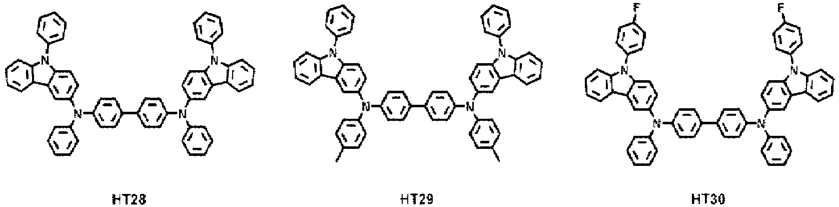

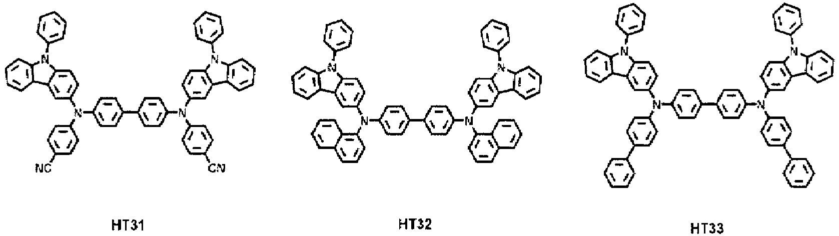

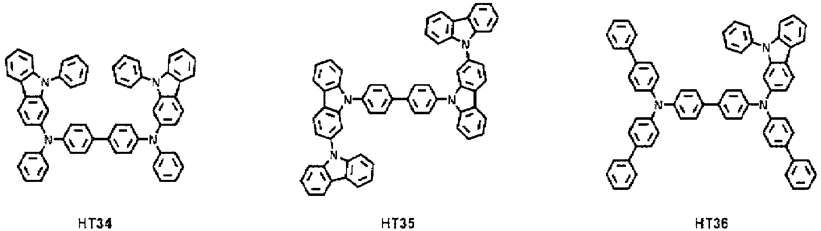

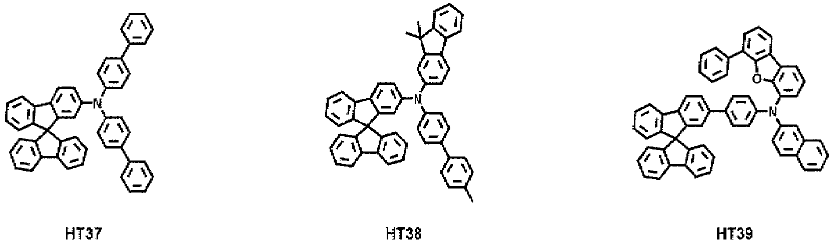

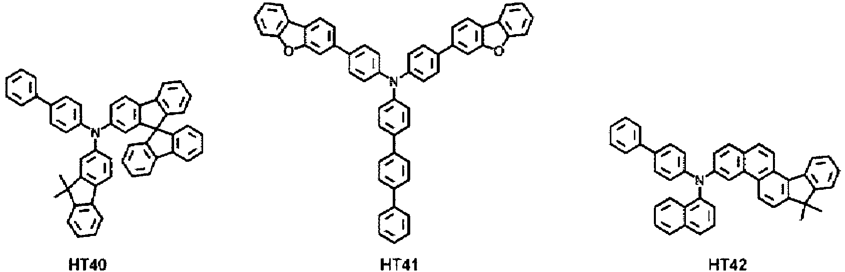

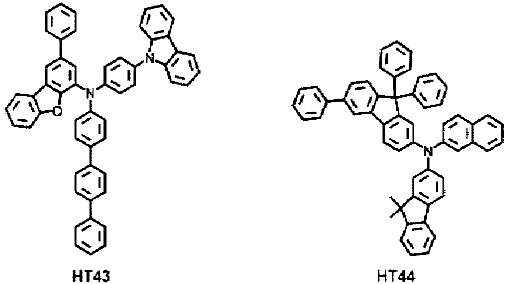

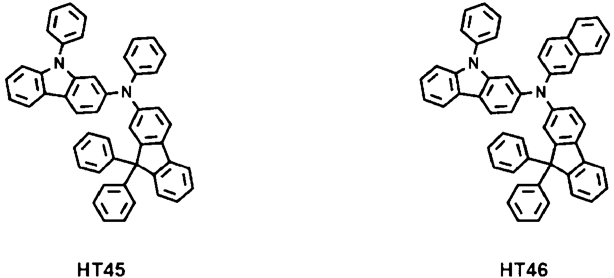

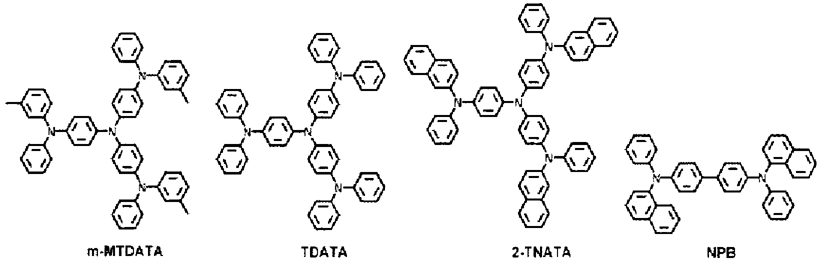

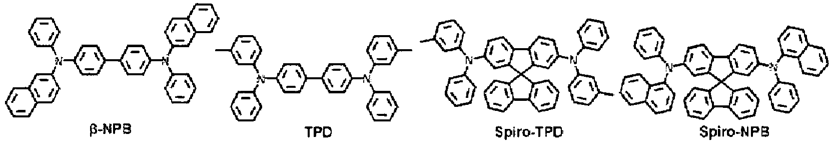

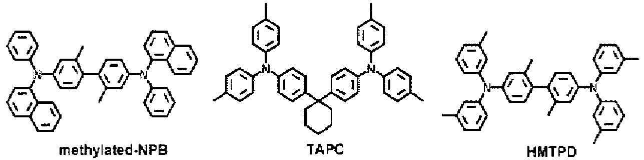

In addition, the hole injection layer material is not particularly limited as long as it is typically used in the art, and may include, for example, 2-TNATA [4,4',4"-tris(2-naphthylphenyl-phenylamino)-triphenylamine], NPD [N,N'-di(1-naphthyl)-N,N'-diphenylbenzidine], TPD [N,N'-diphenyl-N,N'-bis(3-methylphenyl)-1,1'-biphenyl-4,4'-diamine], DNTPD [N,N'-diphenyl-N,N'-bis-[4-(phenyl-m-tolyl-amino)-phenyl]-biphenyl-4,4'-diamine], or HAT-CN [1,4,5,8,9,11-hexaazatriphenylenehexacarbonitrile], although the present disclosure is not limited thereto.

So long as it is commonly used in the art, any hole transport layer material is not particularly limited and may include, for example, N,N'-bis(3-methylphenyl)-N,N'-diphenyl-[1,1-biphenyl]-4,4'-diamine (TPD), N,N'-di(naphthalen-1-yl)-N,N'-diphenylbenzidine (α-NPD), or N-[[1,1'-biphenyl]-4-yl]-9,9-dimethyl-N-[4-(9-phenyl-9H-carbazol-3-yl)phenyl]-9H-fluoren-2-amine (BCFN), although the present disclosure is not limited thereto.

In this case, the thickness of the hole transport region (40) may be about 50 Å to about 10,000 Å, for example, about 100 Å to about 4,000 Å. When the hole transport region (40) includes a hole injection layer, a hole transport layer, or any combination thereof, the thickness of the hole injection layer may be about 100 Å to about 9,000 Å, for example, about 100 Å to about 1,000 Å, and the thickness of the hole transport layer may be about 50 Å to about 2,000 Å, for example, about 100 Å to about 1,500 Å.

Subsequently, a photoactive layer (50, 50') may be stacked on the hole transport region (40) by vacuum deposition or spin coating.

Here, the photoactive layer (50, 50') can absorb incident light and generate an electrical signal. Accordingly, a photodiode including the photoactive layer may function as an optical sensor.

The photoactive layer may include a first layer (50) adjacent to the hole transport region (40) and a second layer (50') adjacent to the electron transport region (60), and, as described above, the organic compound represented by Chemical Formula 1 may be included in the first layer.

In this regard, the first layer in the photoactive layer is in direct contact with the hole transport region, thereby providing advantages of improved external quantum efficiency (EQE), high thermal stability (Td) that prevents easy molecular decomposition, and low dark current density (current flowing in the absence of light irradiation).

According to a specific embodiment of the present disclosure, the thickness of the first layer (50) in the photoactive layer may be 50 Å to 500 Å, preferably 100 Å to 300 Å, and the thickness of the second layer (50') may be 50 Å to 500 Å, preferably 200 Å to 400 Å.

An electron transport region (60) may be deposited on the photoactive layer by vacuum deposition or spin coating.

In the present disclosure, the electron transport region (60) may be disposed on the photoactive layer (50, 50') of the photodiode. The electron transport region (60) may have i) a single-layer structure consisting of a single material, ii) a single-layer structure consisting of a single layer including a plurality of different materials, or iii) a multi-layer structure comprising a plurality of layers including a plurality of different materials.

The electron transport region (60) may include a buffer layer, a hole blocking layer, an electron control layer, an electron transport layer, an electron injection layer, or any combination thereof.

For example, the electron transport region (60) may have a structure such as an electron transport layer/electron injection layer, a hole blocking layer/electron transport layer/electron injection layer, an electron control layer/electron transport layer/electron injection layer, or a buffer layer/electron transport layer/electron injection layer, sequentially stacked from the emission layer.

The electron transport region (for example, the buffer layer, hole blocking layer, electron control layer, or electron transport layer of the electron transport region) may include a metal-free compound including at least one π electron-deficient nitrogen-containing C₁-C₆₀ cyclic group.

The electron transport region may include one or more of the compounds ET1 to ET45, BCP (2,9-dimethyl-4,7-diphenyl-1,10-phenanthroline), Bphen (4,7-diphenyl-1,10-phenanthroline), Alq₃, BAlq, TAZ, NTAZ, or any combination thereof:

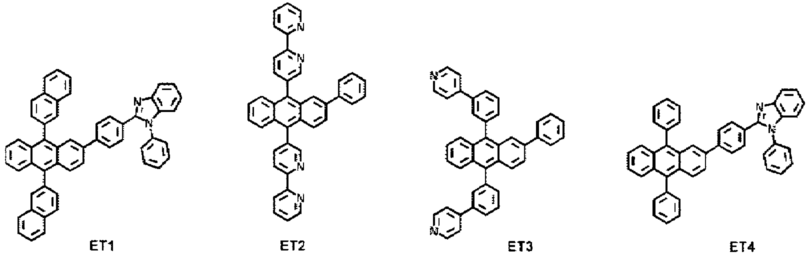

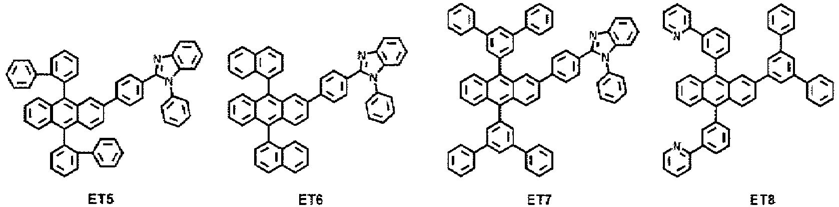

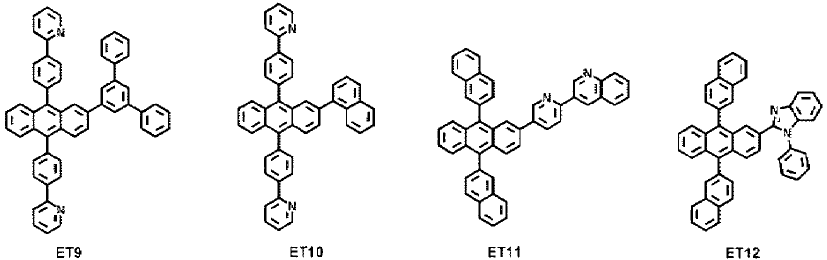

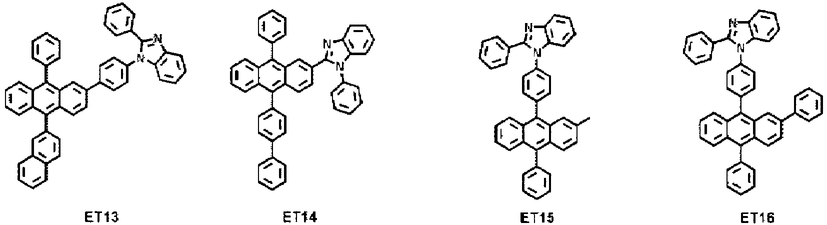

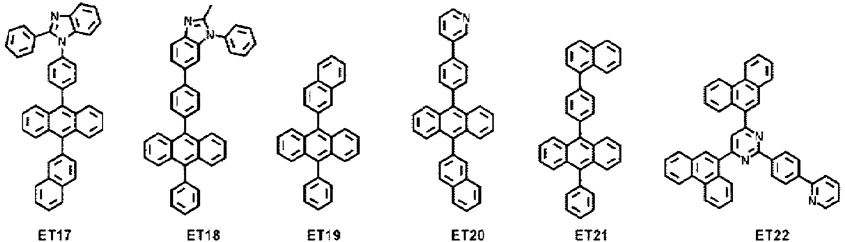

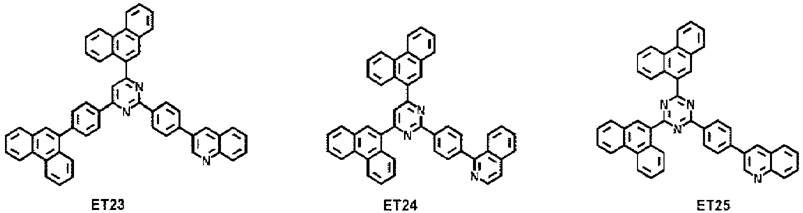

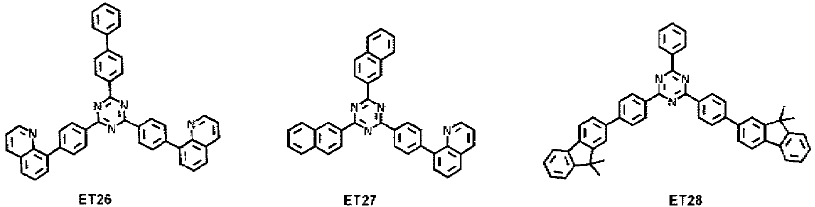

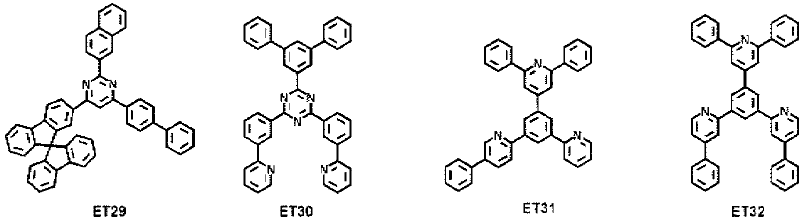

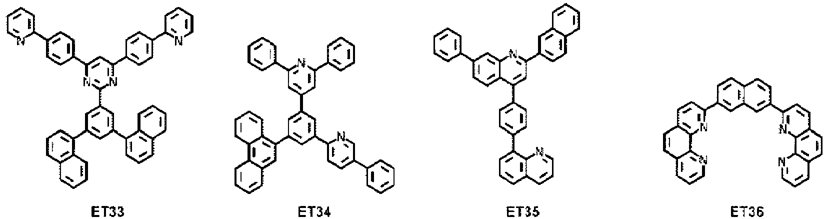

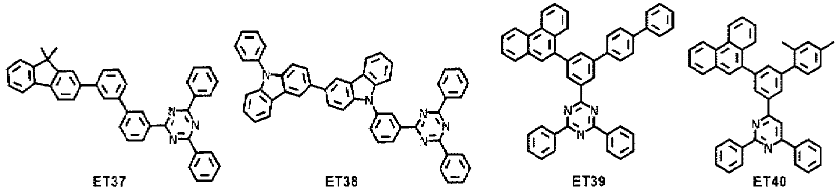

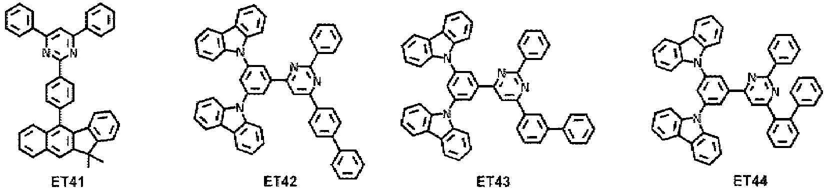

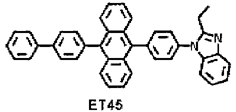

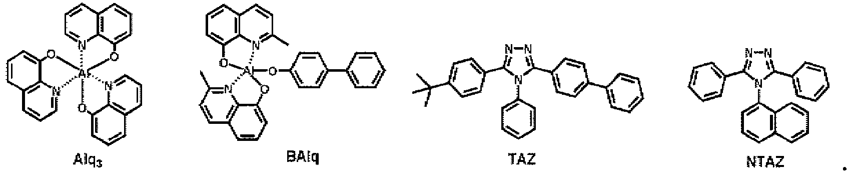

The thickness of the electron transport region may be about 100 Å to about 5,000 Å, for example, about 160 Å to about 4,000 Å. When the electron transport region includes a buffer layer, a hole blocking layer, an electron control layer, an electron transport layer, or any combination thereof, the thicknesses of the buffer layer, the hole blocking layer, or the electron control layer may each independently be about 20 Å to about 1,000 Å, for example, about 30 Å to about 300 Å, and the thickness of the electron transport layer may be about 100 Å to about 1,000 Å, for example, about 150 Å to about 500 Å. When the thicknesses of the buffer layer, the hole blocking layer, the electron control layer, the electron transport layer, and/or the electron transport region satisfy the above-described ranges, satisfactory electron transport properties can be obtained without substantial increase in driving voltage.

The electron transport region (for example, the electron transport layer in the electron transport region) may further include, in addition to the materials described above, a metal-containing material.

The metal-containing material may include an alkali metal complex, an alkaline earth metal complex, or any combination thereof. The metal ion of the alkali metal complex may be a Li ion, Na ion, K ion, Rb ion, or Cs ion, and the metal ion of the alkaline earth metal complex may be a Be ion, Mg ion, Ca ion, Sr ion, or Ba ion. The ligands coordinated to the metal ions of the alkali metal complexes and the alkaline earth metal complexes may each independently include hydroxyquinoline, hydroxyisoquinoline, hydroxybenzoquinoline, hydroxyacridine, hydroxyphenanthridine, hydroxyphenyl-oxazole, hydroxyphenyl-thiazole, hydroxyphenyl-oxadiazole, hydroxyphenyl-thiadiazole, hydroxyphenyl-pyridine, hydroxyphenyl-benzimidazole, hydroxyphenyl-benzothiazole, bipyridine, phenanthroline, cyclopentadiene, or any combination thereof.

For example, the metal-containing material may include a lithium (Li) complex. The lithium complex may include, for example, the compound ET-D1 (Liq) or ET-D2, as described below:

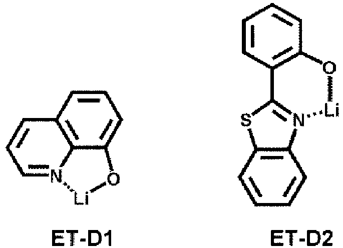

As a material for the electron transport layer, a known electron transport material may be used, which functions to stably transport electrons injected from the electron injection electrode (cathode). Examples of such known electron transport materials include quinoline derivatives, in particular, tris(8-quinolinolato)aluminum (Alq₃), Liq, TAZ, BAlq, beryllium bis(benzoquinolin-10-olate) (Bebq₂), Compound 201, Compound 202, BCP, oxadiazole derivatives such as PBD, BMD, and BND, but are not limited thereto:

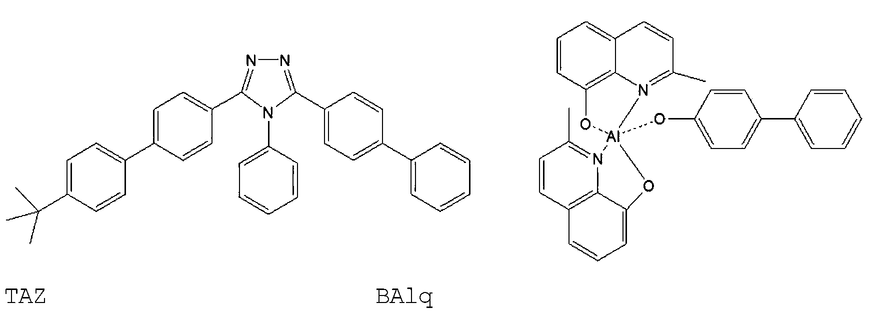

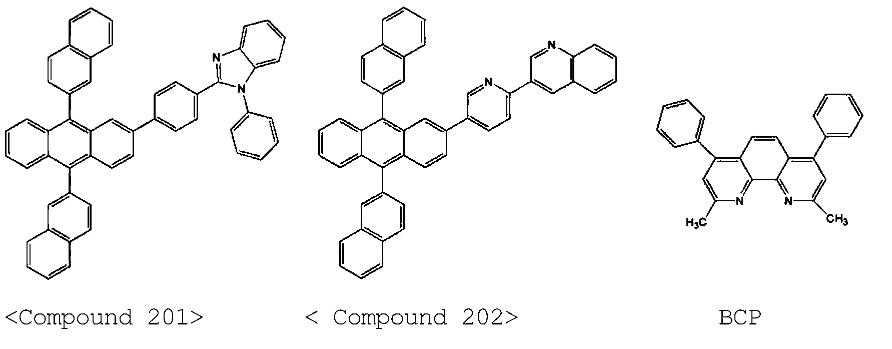

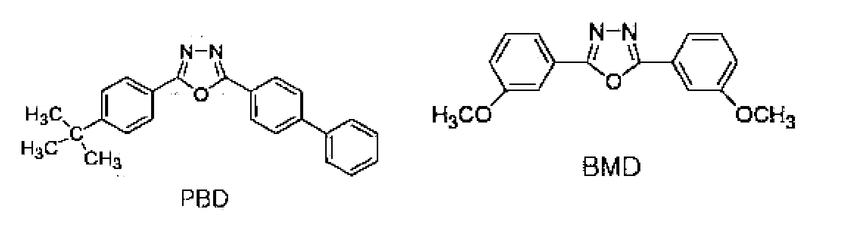

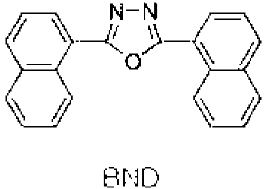

In addition, in the present disclosure, the electron transport region may include an electron injection layer that facilitates electron injection from the second electrode (80). The electron injection layer may be in direct contact with the second electrode (80).

The electron injection layer may have: i) a single-layer structure consisting of a single material, ii) a single-layer structure consisting of a single layer including a plurality of different materials, or iii) a multilayer structure comprising a plurality of layers including a plurality of different materials.

After forming the electron transport layer, an electron injection layer (EIL), which is a material that facilitates electron injection from the cathode, may be further stacked on the upper portion of the electron transport layer, and the material thereof is not particularly limited.

As the material for forming the electron injection layer, any known material may be used, such as CsF, NaF, LiF, Li₂O, or BaO. The deposition conditions of the electron injection layer may vary depending on the compound used, but are generally within substantially the same range of conditions as those for forming the hole injection layer.

The thickness of the electron injection layer may be about 1 Å to about 100 Å, for example, about 3 Å to about 90 Å. When the thickness of the electron injection layer satisfies the above-described ranges, satisfactory electron injection properties can be obtained without substantial increase in driving voltage.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may each include an oxide, a halide (e.g., fluoride, chloride, bromide, iodide, etc.), a telluride, or any combination thereof, of the corresponding alkali metal, alkaline earth metal, or rare earth metal.

The alkali metal-containing compound may include alkali metal oxides such as Li₂O, Cs₂O, and K₂O; alkali metal halides such as LiF, NaF, CsF, KF, LiI, NaI, CsI, and KI; or any combination thereof. The alkaline earth metal-containing compound may include alkaline earth metal compounds such as BaO, SrO, CaO, BaₓSr₁₋ₓO (where x is a real number satisfying 0 < x < 1), or BaₓCa₁₋ₓO (where x is a real number satisfying 0 < x < 1).

The electron injection layer may consist solely of the above-described alkali metal, alkaline earth metal, rare earth metal, alkali metal-containing compound, alkaline earth metal-containing compound, rare earth metal-containing compound, alkali metal complex, alkaline earth metal complex, rare earth metal complex, or any combination thereof, or may further include an organic material.

According to an embodiment, the electron injection layer may i) consist of an alkali metal-containing compound (for example, an alkali metal halide), or ii) consist of a) an alkali metal-containing compound (for example, an alkali metal halide); and b) an alkali metal, an alkaline earth metal, a rare earth metal, or any combination thereof. For example, the electron injection layer may be a co-deposited layer of KI:Yb, RbI:Yb, or LiF:Yb.

The thickness of the electron injection layer may be about 1 Å to about 100 Å, for example, about 3 Å to about 90 Å. When the thickness of the electron injection layer satisfies the above-described ranges, satisfactory electron injection characteristics can be obtained without substantial increase in driving voltage.

In the present disclosure, the second electrode (80) may be disposed on the electron transport region (60). The second electrode (80) may be a cathode serving as an electron injection electrode, and in this case, a material having a low work function may be used as the material of the second electrode (80), such as a metal, an alloy, an electrically conductive compound, or any combination thereof.

The second electrode (80) may include lithium (Li), silver (Ag), magnesium (Mg), aluminum (Al), aluminum-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode (80) may be a transparent electrode, a semitransparent electrode, or a reflective electrode.

The second electrode (80) may have a single-layer structure consisting of one layer, or a multi-layer structure comprising a plurality of layers.

In the present disclosure, when the second electrode (80) is a cathode, a material with a low work function may be used to facilitate electron injection. Lithium (Li), magnesium (Mg), calcium (Ca), or alloys thereof such as aluminum (Al), aluminum-lithium (Al-Li), magnesium-indium (Mg-In), or magnesium-silver (Mg-Ag) may be used, or alternatively, a transparent cathode formed of ITO or IZO may be used.

In addition, the photodiode according to the present disclosure may further include a capping layer (not shown) on the outer surface of the first electrode or the second electrode.

For example, a first capping layer may be disposed on the outside of the first electrode (20), and/or a second capping layer may be disposed on the outside of the second electrode (80).

The first capping layer and the second capping layer may serve to improve external light emission efficiency by the principle of constructive interference.

Each of the first capping layer and the second capping layer may comprise a material having a refractive index of 1.6 or higher (at 589 nm).

The first capping layer and the second capping layer may independently be an organic capping layer comprising an organic material, an inorganic capping layer comprising an inorganic material, or an organic-inorganic composite capping layer comprising both an organic material and an inorganic material.

At least one of the first capping layer and the second capping layer may independently include a carbocyclic compound, a heterocyclic compound, an amine group-containing compound, porphine derivatives, phthalocyanine derivatives, naphthalocyanine derivatives, an alkali metal complex, an alkaline earth metal complex, or a rare earth metal complex, or any combination thereof. The carbocyclic compound, heterocyclic compound, and amine group-containing compound may optionally be substituted with a substituent including O, N, S, Se, Si, F, Cl, Br, I, or any combination thereof. According to an embodiment, at least one of the first capping layer and the second capping layer may independently include an amine group-containing compound.

Below, a better understanding of the present disclosure may be obtained through the following examples which are set forth to illustrate, but are not to be construed to limit, the scope of the present invention.

### EXAMPLES

### SYNTHESIS EXAMPLE 1. Synthesis of Compound <1>

### SYNTHESIS EXAMPLE 1. Synthesis of <1>

### Synthesis Example 1-1. Synthesis of <1-a>

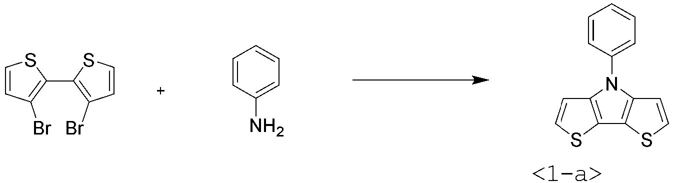

In a 500 mL round-bottom flask, 3,3'-dibromo-2,2'-bithiophene (20 g), aniline (6.9 g), tris(dibenzylideneacetone)dipalladium(0) (1.7 g), sodium tert-butoxide (18 g), 2,2'-bis(diphenylphosphino)-1,1'-binaphthyl (4.6 g), and toluene (200 mL) were together stirred under reflux. After completion of the reaction, the reaction mixture was cooled to room temperature. Extraction with ethyl acetate and water was conducted to separate the organic layer which was then concentrated in a vacuum. Purification through silica gel column chromatography afforded <1-a>. (12 g, 76%)

### Synthesis Example 1-2. Synthesis of <1-b>

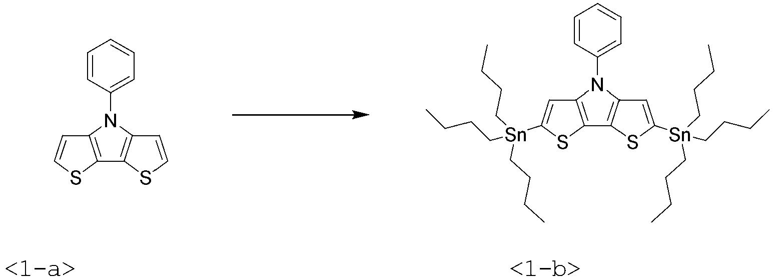

In a 250 mL round-bottom flask, a mixture of <1-a> (12 g) and tetrahydrofuran (120 mL) was chilled to -78°C under a nitrogen atmosphere. To the chilled reaction solution was dropwise added n-butyl lithium (40 mL) at the same temperature. After stirring for 2 hours, tributyltin chloride (28 mL) was added little by little. The reaction mixture was stirred again for 12 hours at room temperature and subjected to extraction with ethylacetate and water. The organic layer thus formed was concentrated in a vacuum to afford <1-b>. (35 g, 90%)

### Synthesis Example 1-3. Synthesis of <1-c>

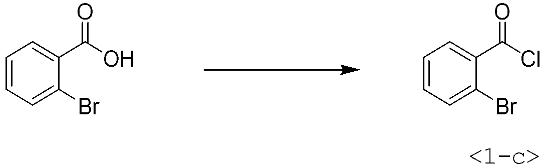

In a 250 mL round-bottom flask reactor, a solution of 2-bromobenzoic acid (30 g) in toluene (200 mL) was added with thionyl chloride (53 g) at room temperature, followed by dimethylform amide (2 mL). The reaction mixture was stirred until it was completely dissolved. Concentration in a vacuum afforded <1-c>. (32 g, 97%)

### Synthesis Example 1-4. Synthesis of <1-d>

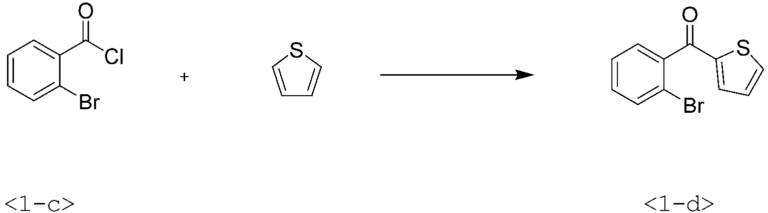

In a 250 mL round-bottom flask, a solution of aluminum chloride (15 g) in dichloromethane (90 mL) was cooled to 0°C under a nitrogen atmosphere. After 15 minutes, <1-c> (32 g) was dropwise added to the cold reaction solution at the same temperature. Addition of drops of thiophene (9 g) was followed by stirring at room temperature. The reaction was terminated by adding water (50 mL). Extraction with dichloromethane gave an organic layer which was then concentrated in a vacuum. Purification by column chromatography afforded <1-d>. (28 g, 93%)

### Synthesis Example 1-5. Synthesis of <1-e>

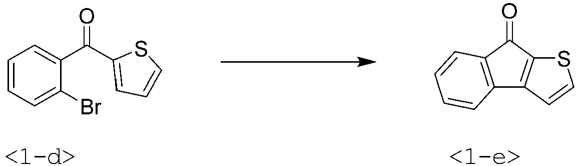

In a 100 mL round-bottom flask, a mixture of <1-d> (28 g), palladium acetate (1.2 g), tricyclohexylphosphonium tetrafluoroborate (7.7 g), potassium carbonate (36 g), and dimethylformamide (280 mL) was stirred at 130°C. After completion of the reaction, extraction with dichloromethane and water gave an organic layer which was then concentrated in a vacuum. Purification by column chromatography afforded <1-e>. (8 g, 41%)

### Synthesis Example 1-6. Synthesis of <1-f>

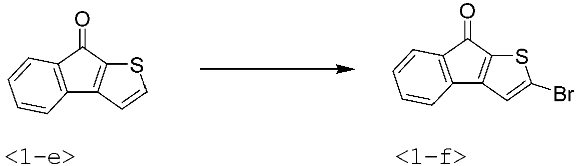

In a 100 mL round-bottom flask, a mixture of <1-e> (8 g) and dimethylformamide (80 mL) was cooled to 0°C. A solution of N-bromosuccinimide in dimethylformamide was dropwise added to the chilled reactor, followed by temperature elevation to room temperature. After completion of the reaction, water (100 mL) was poured to the reaction mixture. The solid thus formed was filtered, washed with water, and dried to afford <1-f>. (6.8 g, 75%)

### Synthesis Example 1-7. Synthesis of <1>

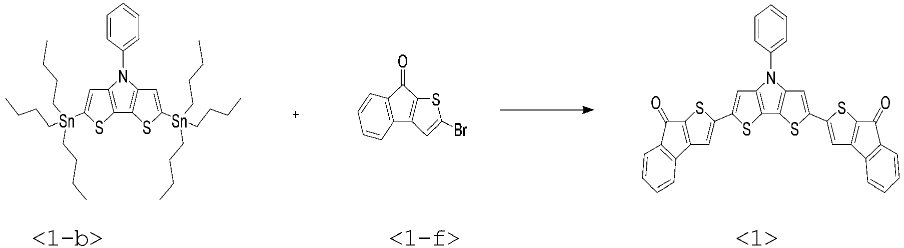

In a 250 mL round-bottom flask, a mixture of <1-b> (9 g), <1-e> (6 g), tetrakis(triphenylphosphine)palladium (0.7 g), and toluene (180 mL) was stirred under reflux in a nitrogen atmosphere. After completion of the reaction, the reaction mixture was cooled to room temperature. Addition of methanol was followed by stirring. The solid thus formed was filtrated. The filtrate was added to acetone, heated, and then filtered. Washing with acetone and drying afforded <1>. (3.2 g, 48%)
MS (MALDI-TOF) : m/z 623.01 [M+]

### SYNTHESIS EXAMPLE 2. Synthesis of <11>

### Synthesis Example 2-1. Synthesis of <2-a>

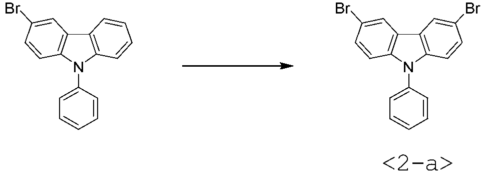

In a 500 mL round-bottom flask reactor, a mixture of 3-bromo-9-phenyl-9H-carbazole (30 g) and dimethylformamide (200 mL) was cooled to 0°C. A solution of N-bromosuccinimide (25 g) in dimethylformamide (100 mL) was dropwise added to the cooled reactor and then warmed to room temperature. After completion of the reation, water (300 mL) was poured to the reaction mixture. The solid thus formed was filtered, washed with water, and dried to afford <2-a>. (36 g, 96%)

### Synthesis Example 2-2. Synthesis of <2-b>

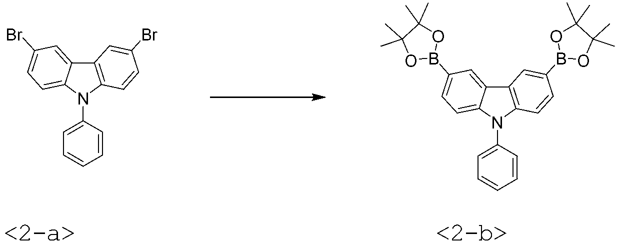

In a 250 mL round-bottom flask, <2-a> (10 g), a mixture of bis(pinacolate)diboron 25 g, dichloro[1,1'-bis (diphenylphosphino)ferrocene] palladium (1.8 g), potassium acetate (17 g), and toluene (100 mL) was stirred under reflux. After completion of the reaction, the reaction mixture was subjected to extraction with ethylacetate and water. The organic layer thus formed was separated, concentrated in a vacuum, and purified by column chromatography to afford <2-b>. (7.5 g, 60%)

### Synthesis Example 2-3. Synthesis of <2-c>

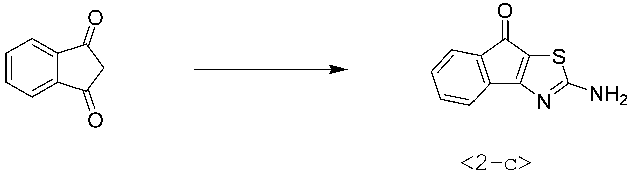

In a 500 mL round-bottom flask, a mixture of 1,3-indanedione (20 g) and ethanol (200 mL) was cooled to 0°C. To the cold mixture was dropwise added N-bromosuccinimide (48 g), followed by stirring for 2 hours. Thiourea (20 g) and dimethylformamide (20 g) were added before stirring for 6 hours under reflux. After completion of the reaction, the reaction mixture was cooled to room temperature. The solid thus formed was filtered and recrystallized in ethanol to afford <2-c>. (15 g, 55%)

### Synthesis Example 2-4. Synthesis of <2-d>

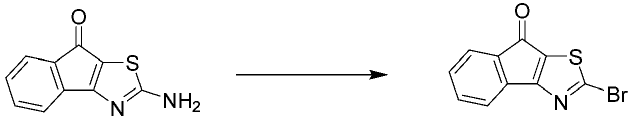

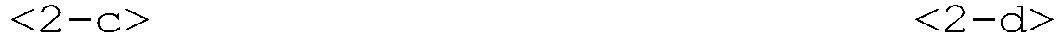

In a 500 mL round-bottom flask, copper bromide (26 g) and acetonitrile (300 mL) were stirred together. Subsequently, tert-butyl nitride (23 g was added and stirred at 60°C for 10 minutes. To the mixture was added
<2-c> (15 g), followed by stirring at the same temperature for 1 hour. After completion of the reaction, the reaction mixture was cooled to room temperature. 1 N HCl was added and then extraction with dichloromethane was carried out. The organic layer thus formed was separated, concentrated in a vacuum, and purified by column chromatography to afford <2-d>. (4 g, 20 %)

### Synthesis Example 2-5. Synthesis of <11>

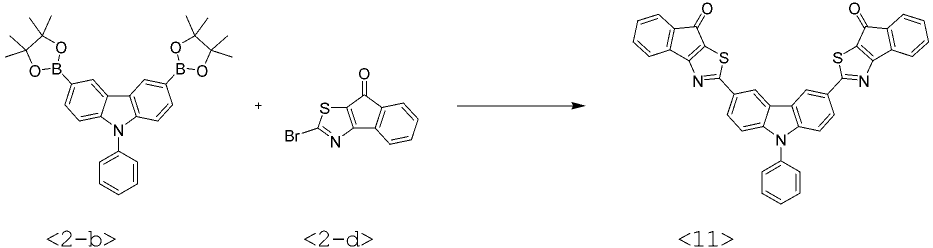

In a 100 mL round-bottom flask, a mixture of <2-b> (7.4 g), <2-d> (8.6 g), potassium phosphate (16 g), tetrakis(triphenylphosphine)palladium (1.7 g), toluene (60 mL), ethanol (15 mL), and water (15 mL) was stirred under reflux. After completion of the reaction, the reaction mixture was cooled to room temperature. The red solid thus formed was washed with methanol and then completely dissolved in dichlorobenzene (1500 mL), followed by filtration through silica gel. Recrystallization in dichlorobenzene and acetone afforded <11>. (8.4 g, 93%)
MS (MALDI-TOF) : m/z 611.10 [M+]

### SYNTHESIS EXAMPLE 3. Synthesis of <16>

### Synthesis Example 3-1. Synthesis of <3-a>

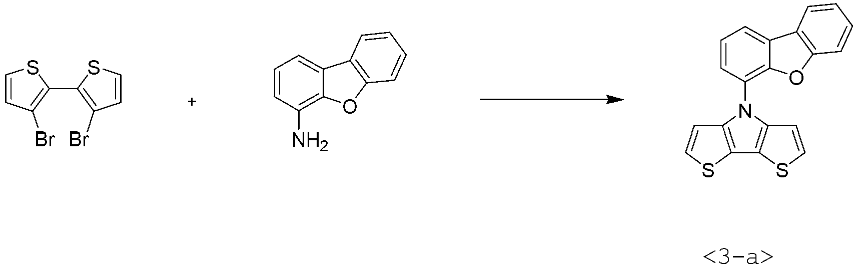

The same manner as in Synthesis Example 1-1, with the exception of using dibenzo[B,D]furan-4-amine instead of aniline, was carried out to afford <3-a>. (yield 60%)

### Synthesis Example 3-2. Synthesis of <3-b>

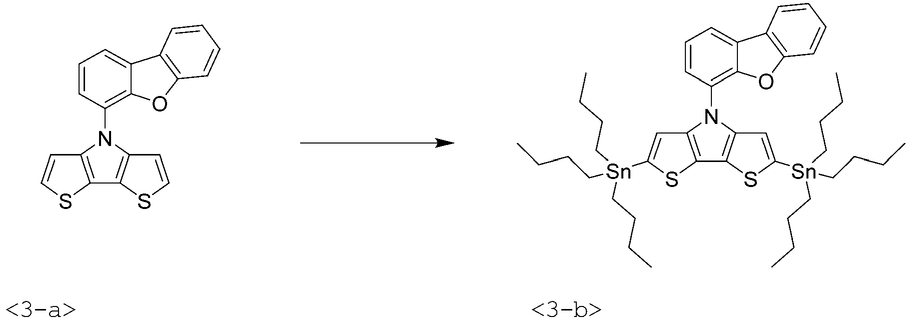

The same manner as in Synthesis Example 1-2, with the exception of using <3-a> instead of <1-a>, was carried out to afford <3-b>. (yield 90%)

### Synthesis Example 3-3. Synthesis of <3-c>

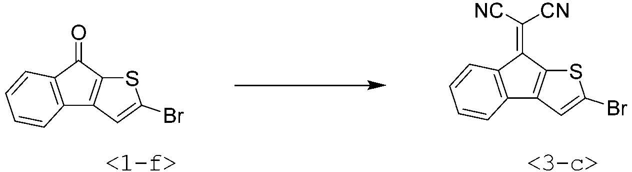

In a 100 mL round-bottom flask, a mixture of <1-f> (6 g), malononitrile (3 g), sodium acetate (2.5 g), and ethanol (60 mL) was stirred under reflux. After completion of the reaction, the reaction mixture was cooled to room temperature. The solid thus formed was filtered, washed with ethanol, and dried to afford <3-c>. (4 g, 56%)

### Synthesis Example 3-4. Synthesis of <16>

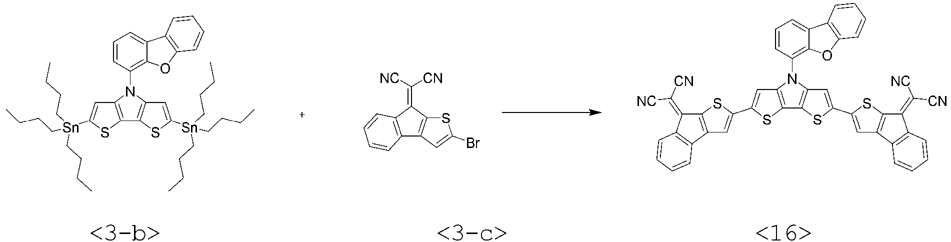

The same manner as in Synthesis Example 1-7, with the exception of using <3-b> and <3-c> instead of <1-b> and <1-e>, respectively, was carried out to afford <16>. (yield 35%)
MS (MALDI-TOF) : m/z 809.05 [M+]

### EXAMPLE: Fabrication of Organic Photodiode

An ITO glass substrate was patterned to have a translucent area of 2 mm x 2 mm and cleansed. The ITO glass was mounted in a vacuum chamber that was then set to have a base pressure of 1x10⁻⁷ torr. On the ITO glass substrate, a hole injection layer was formed (100 Å) by depositing [Compound A] as an electron acceptor and [HT] at a deposition ratio of [Compound A] : [HT] = 3 : 97. Subsequently, [HT] was deposited (1500 Å) as a hole transport layer.

Next, a photoactive layer was constructed by depositing a donor material prepared according to the present disclosure as a first layer (140 Å) and then the acceptor material [Acceptor] described below as a second layer (340 Å). Subsequently, [HB] was deposited (50 Å) as a hole blocking layer, followed by deposition of [ET] and [Liq] in a 1:1 ratio to a thickness of 300 Å as the electron transport layer, [Liq] (10 Å) as the electron injection layer, and MgAg (100 Å), in this order. Thereafter, [CPL] was deposited (600 Å) as a capping layer to fabricate an organic photodiode device. The organic photodiode was evaluated for dark current density and EQE at -3 V, and the results are shown in Table 1 below.

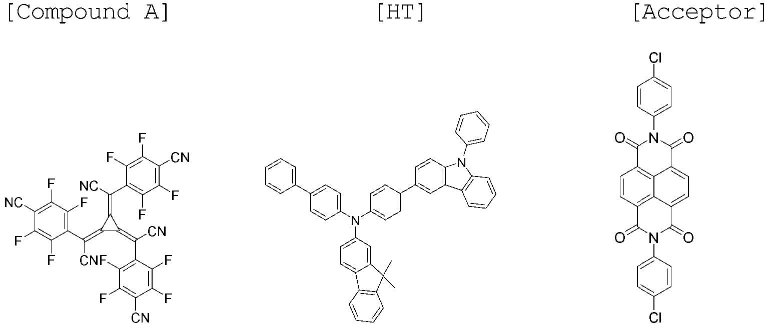

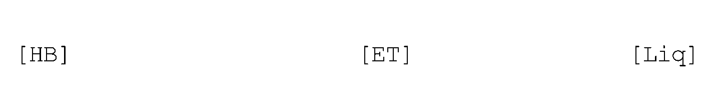

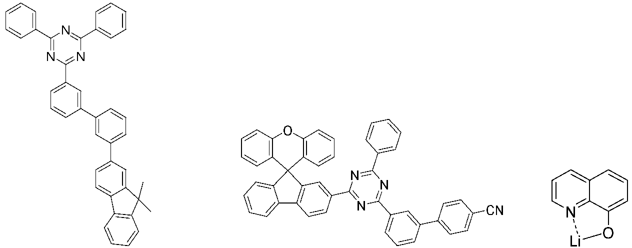

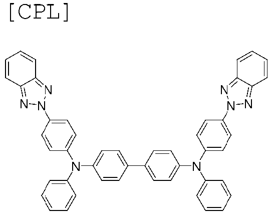

### COMPARATIVE EXAMPLES 1 AND 2

Organic photodiodes of Comparative Examples 1 and 2 were fabricated in the same manner as in the Example, with the exception of using compounds [OPD-A] and [OPD-B] instead of the compounds prepared according to the present disclosure. Structures of [OPD-A] and [OPD-B] are as follows:

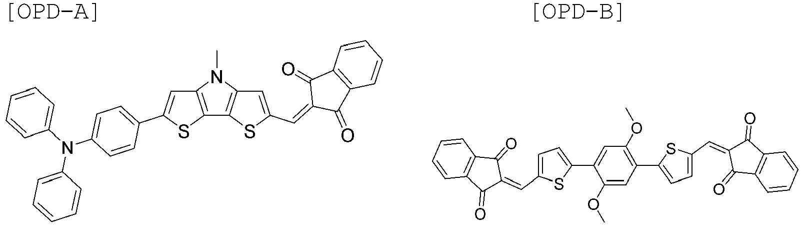

**TABLE 1**

| | Donor | Td [°C] | Dark current density [mA/cm²] | EQE [ % ] |
|---|---|---|---|---|
| Ex. 1 | Compound 1 | 434 | 7.6E-06 | 35 |
| Ex. 2 | Compound 11 | 412 | 7.2E-06 | 32 |
| Ex. 3 | Compound 16 | 386 | 6.5E-06 | 28 |
| C. Ex. 1 | OPD-A | 326 | 3.5E-05 | 6 |
| C. Ex. 2 | OPD-B | 375 | 6.8E-06 | 12 |

### Absorption Characteristics of the Compounds

The absorption characteristics (maximum absorption wavelength) of the compounds according to Examples 1 to 3 and Comparative Examples 1 and 2 were evaluated depending on wavelength. Solutions of each compound in toluene were prepared at a concentration of about 8 × 10⁻⁴ wt%, and thin films with a thickness of 300 Å were also formed. The absorption characteristics of both the toluene solution and the thin film were measured in the ultraviolet-visible (UV-Vis) region, and the results are shown in Table 2 below.

**TABLE 2**

| Compound | Wavelength [nm] (toluene solution) | Wavelength [nm] (thin film) |
|---|---|---|
| Ex. 1 | 550 | 630 |
| Ex. 2 | 457 | 530 |
| Ex. 3 | 670 | 750 |
| C. Ex. 1 | 567 | 604 |
| C. Ex. 2 | 530 | 545 |

As shown in Tables 1 and 2, the organic photodiodes including the compounds represented by Chemical Formula 1 according to the present disclosure exhibited a higher Td value and a higher EQE value compared with the organic photodiodes including the comparative compounds (Comparative Examples 1 and 2) of the related art. In addition, it was confirmed that the compound exhibited absorption characteristics in the wavelength region of 457 to 670 nm in toluene solution and in the wavelength range of 530 to 750 nm as a thin film, indicating high applicability.

## Claims

1. An organic compound represented by the following Chemical Formula 1:
[Chemical Formula 1] A₁(L₁)ₘ-D-(L₂)ₙ-A₂
wherein,
L₁ and L₂, which are same and different, are each independently are any one selected from a single bond, a substituted or unsubstituted arylene of 6 to 18 carbon atoms, a substituted or unsubstituted aliphatic hydrocarbon ring-fused arylene of 8 to 20 carbon atoms, and a substituted or unsubstituted heteroarylene of 2 to 10 carbon atoms,
m and n, which are same or different, are each independently an integer of 1 or 2,
wherein when m is 2, the corresponding L₁'s are same or different, and
when n is 2, the corresponding L₂'s are same or different,
D is a moiety represented by the following Structural Formula 2,
A₁ and A₂, which are same or different, are moieties each represented by the following Structural Formula 3: 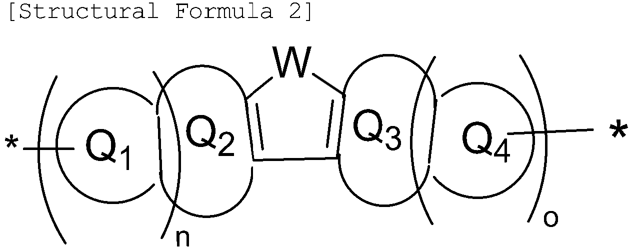 in Structural Formula 2,
W is NR₁ or CR₂R₃,
Q1 to Q4 rings, which are same or different, are each independently any one selected from a substituted or unsubstituted aromatic hydrocarbon ring of 6 to 50 carbon atoms and a substituted or unsubstituted heteroaromatic ring of 2 to 50 carbon atoms,
n and o, which are same or different, are each independently an integer of 0 to 2,
wherein when n is 2, the corresponding Q1 rings are same or different, and
when o is 2, the corresponding Q4 rings are same or different,
R₁ is any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted aromatic hydrocarbon ring-fused cycloalkyl of 7 to 30 carbon atoms, a substituted or unsubstituted aliphatic hydrocarbon ring-fused aryl of 8 to 30 carbon atoms, and a substituted or unsubstituted heteroaryl of 2 to 50 carbon atoms,
R₂ and R₃, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted aromatic hydrocarbon ring-fused cycloalkyl of 7 to 30 carbon atoms, and a substituted or unsubstituted aliphatic hydrocarbon ring-fused aryl of 8 to 30 carbon atoms and can be connected to each other to form a ring, 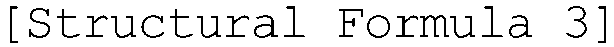 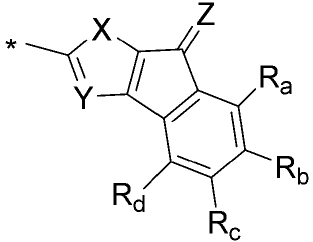 in Structural Formula 3,
X is S or O,
Y is any one selected from CH, CD, and N,
Z is O or malononitrile(=C(CN)₂),
Ra to Rd, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted amine of 0 to 40 carbon atoms, a substituted or unsubstituted silyl of 0 to 40 carbon atoms, a nitro, a cyano, and a halogen,
in Structural Formulas 2 and 3, "*" means a bonding site to which the linker L₁ or L₂ in Chemical Formula 1 is bonded,
wherein the term "substituted" in the expression "a substituted or unsubstituted" used for the compounds of Chemical Formula 1 and Structural Formulas 2 and 3 means having at least one substituent selected from the group consisting of a deuterium atom, a tritium atom, a cyano, a halogen, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.

2. The organic compound of claim 1, wherein Q1 to Q4 rings in Structural Formula 2, which are same or different, are each independently any one selected from a substituted or unsubstituted thiophene ring, a substituted or unsubstituted furan ring, a substituted or unsubstituted benzene ring, a substituted or unsubstituted benzothiophene ring, a substituted or unsubstituted benzofuran ring, and a substituted or unsubstituted indole ring.

3. The organic compound of claim 2, wherein the compound represented by Structural Formula 2 is a compound represented by the following Structural Formulas 2a and 2c: 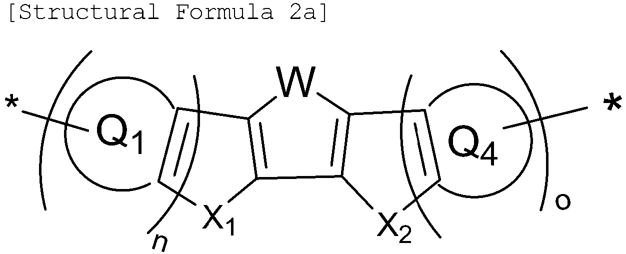 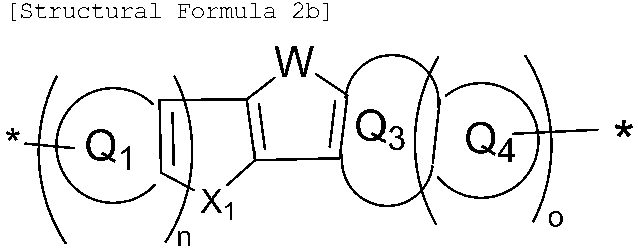 wherein,
X₁ and X₂, which are same or different, are each independently any one selected from S, O, and NR,
R is any one selected from a hydrogen atom, a deuterium atom, a tritium atom, a substituted or unsubstituted alkyl of 1 to 30 carbon atoms, a substituted or unsubstituted cycloalkyl of 3 to 30 carbon atoms, a substituted or unsubstituted aryl of 6 to 50 carbon atoms, a substituted or unsubstituted aliphatic hydrocarbon ring-fused aryl of 8 to 30 carbon atoms, and a substituted or unsubstituted heteroaryl of 2 to 50 carbon atoms,
Q3 ring in Structural Formula 2b is a substituted or unsubstituted benzene ring, and
W, Q1, Q4, n and o are each as defined in Structural Formula 2.

4. The organic compound of claim 2, wherein Q2 and Q3 rings in Structural Formula 2 are each a substituted or unsubstituted benzene ring.

5. The organic compound of claim 1, wherein at least one hydrogen atom in Chemical Formula 1 is substituted with a deuterium atom or a tritium atom.

6. The organic compound of claim 1, wherein W in Structural Formula 2 is NR₁, wherein R₁ is any one substituent selected from a substituted or unsubstituted alkyl of 1 to 20 carbon atoms, a substituted or unsubstituted halogenated alkyl of 1 to 20 carbon atoms, a substituted or unsubstituted aryl of 6 to 20 carbon atoms, a substituted or unsubstituted cycloalkyl of 3 to 20 carbon atoms, a substituted or unsubstituted aromatic hydrocarbon ring-fused cycloalkyl of 8 to 20 carbon atoms, and a substituted or unsubstituted aliphatic hydrocarbon ring-fused aryl of 9 to 20 carbon atoms.

7. The organic compound of claim 1, wherein o in Structural Formula 2 is 0.

8. The organic compound of claim 1, wherein the Q3 ring in Structural Formula 2 is a substituted or unsubstituted thiophene ring or a substituted or unsubstituted furan ring.

9. The organic compound of claim 1, wherein the compound represented by Chemical Formula 1 is any one selected from the following Compounds 1 to 39:

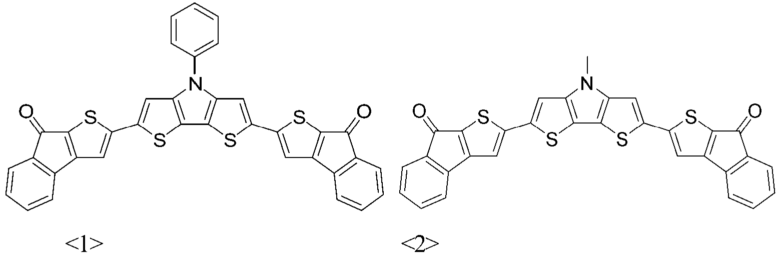

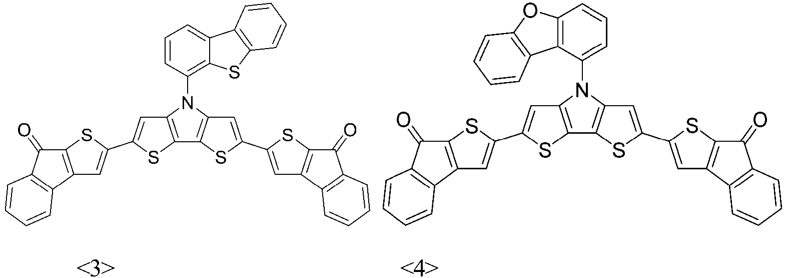

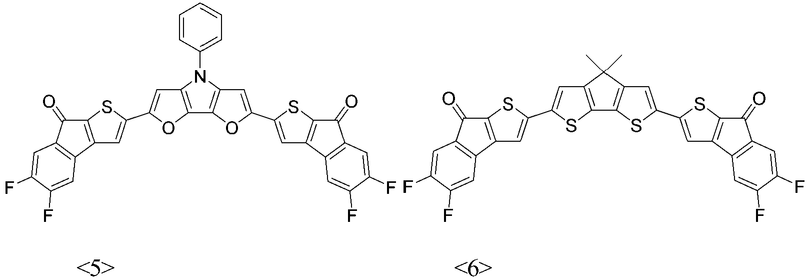

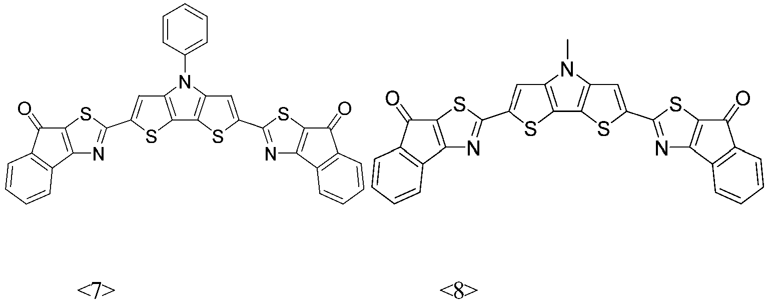

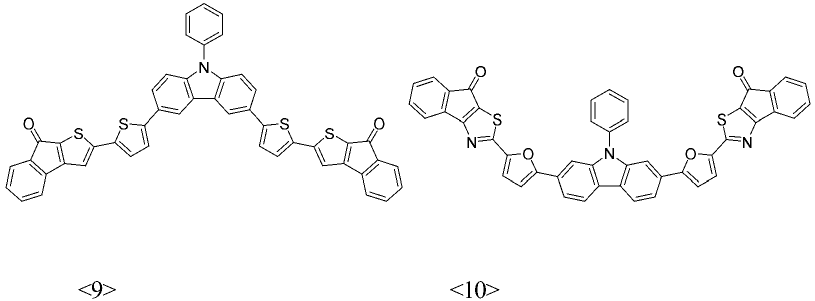

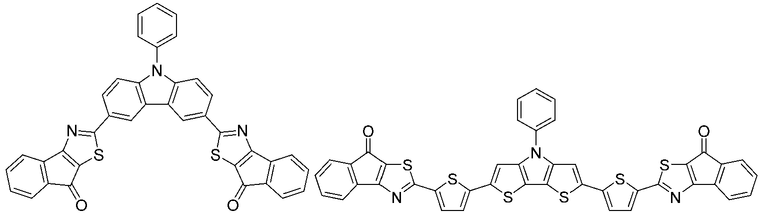

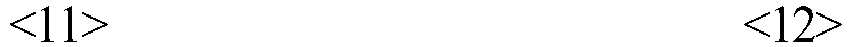

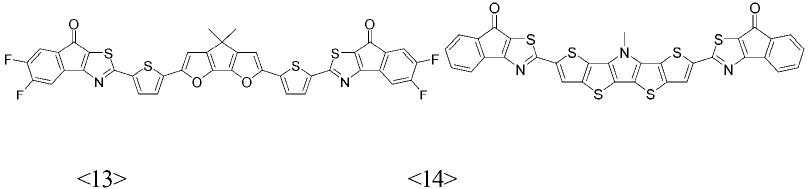

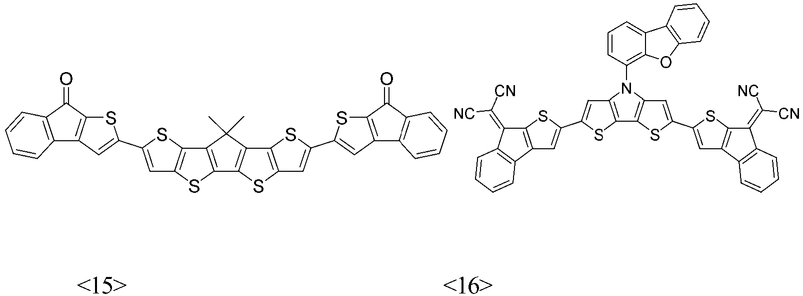

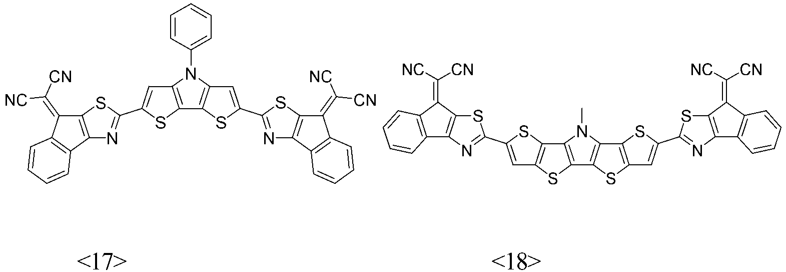

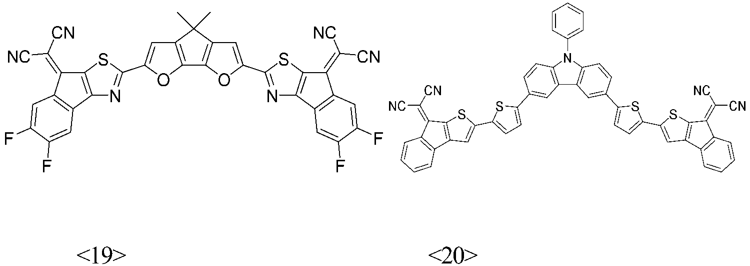

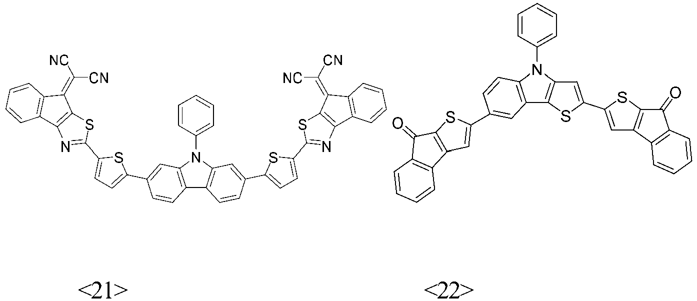

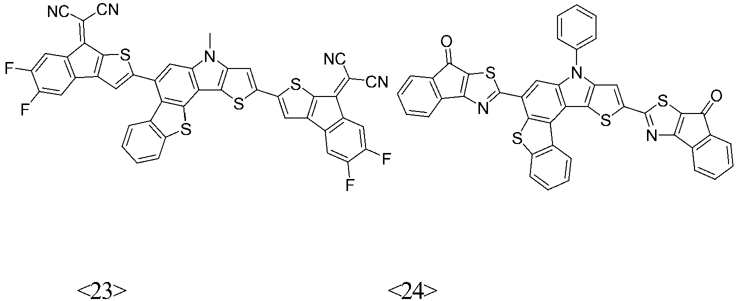

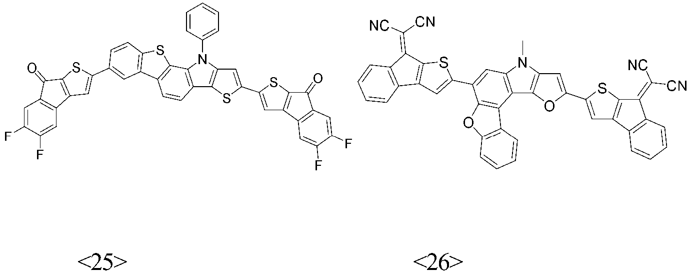

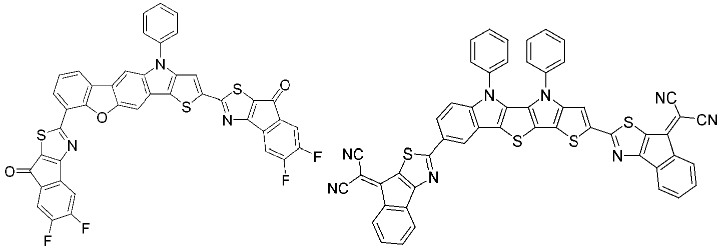

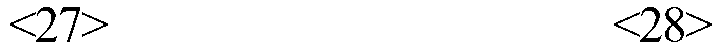

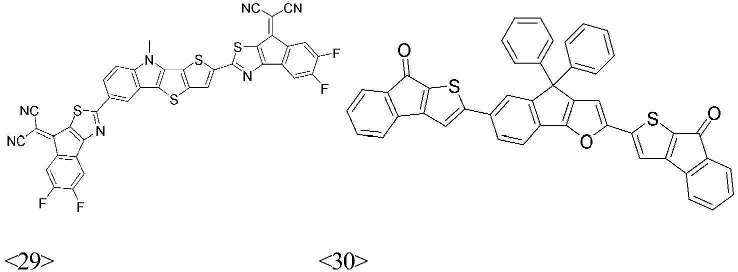

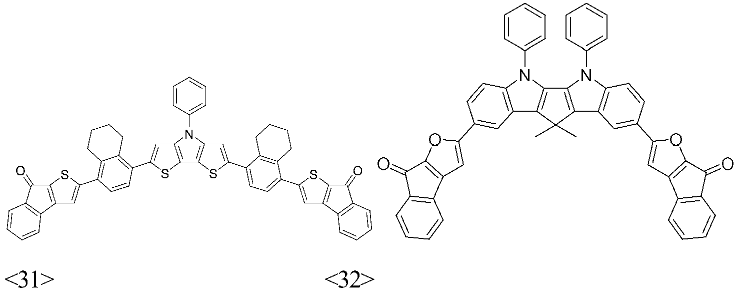

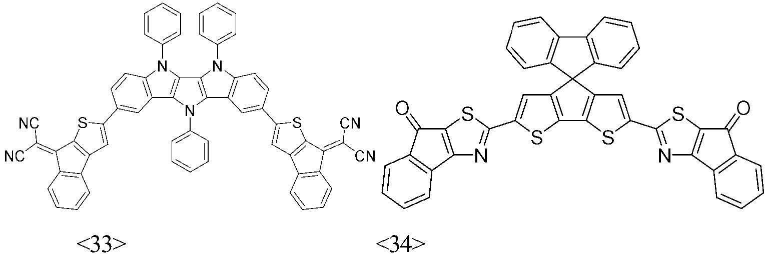

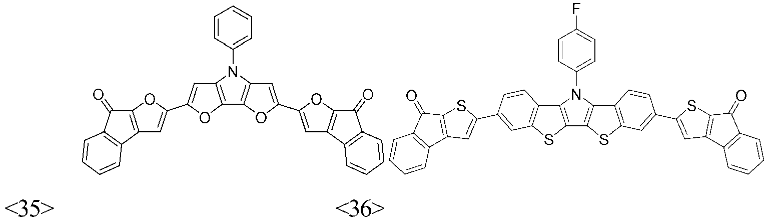

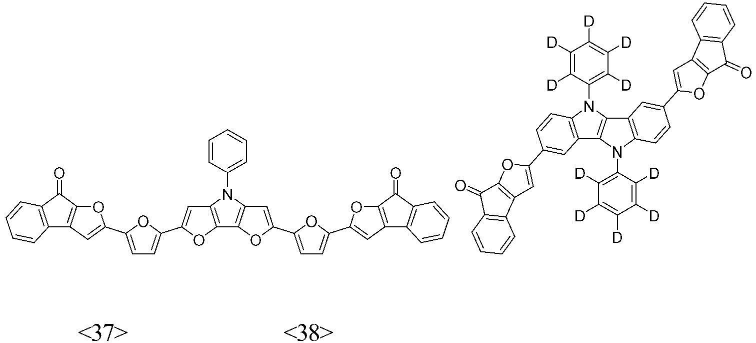

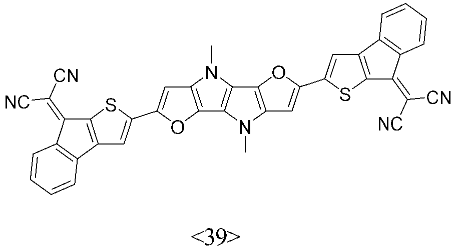

10. A photodiode, comprising:
a first electrode;
a second electrode facing the first electrode; and
a photoactive layer disposed between the first electrode and the second electrode,
wherein the photoactive layer comprises an organic compound represented by Chemical Formula 1 of any one of claims 1 to 9.

11. The photodiode of claim 10, further comprising a hole transport region disposed between the first electrode and the photoactive layer and an electron transport region disposed between the photoactive layer and the second electrode.

12. The photodiode of claim 11, wherein the photoactive layer comprises a first layer adjacent to the hole transport region and a second layer adjacent to the electron transport region, and the organic compound is contained in the first layer.

13. The photodiode of claim 12, wherein the first layer in the photoactive layer is in direct contact with the hole transport region.

14. The electronic apparatus, comprising the photodiode of claim 10.

15. The electronic apparatus of claim 14, further comprising:
a thin film transistor electrically connected to the first electrode; and
an emission layer disposed between the first electrode and the second electrode.
